(19) Europäisches Patentamt
European Patent Office
Office européen des brevets

(11) EP 3 076 175 A1

(12) **EUROPEAN PATENT APPLICATION**

(43) Date of publication:
**05.10.2016 Bulletin 2016/40**

(51) Int Cl.:
***G01N 33/50*** *(2006.01)* ***C12Q 1/34*** *(2006.01)*

(21) Application number: **15162317.0**

(22) Date of filing: **02.04.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Boehringer Ingelheim International GmbH**
**55216 Ingelheim am Rhein (DE)**

(72) Inventors:
- **BUETTNER, Frank**
  **55216 Ingelheim am Rhein (DE)**
- **SCHNAPP, Gisela**
  **55216 Ingelheim am Rhein (DE)**
- **DUESSEL, René**
  **55216 Ingelheim am Rhein (DE)**
- **FIEGEN, Dennis**
  **55216 Ingelheim am Rhein (DE)**
- **WALTER, Rainer**
  **55216 Ingelheim am Rhein (DE)**

(74) Representative: **Simon, Elke Anna Maria et al**
**Boehringer Ingelheim GmbH**
**Binger Strasse 173**
**55216 Ingelheim am Rhein (DE)**

# (54) DETECTION AND QUANTIFICATION OF O-ACETYL-ADP-RIBOSE AS A READOUT SYSTEM FOR MEASURING THE ACTIVITY OF E.G. SIRTUINS

(57) The present invention provides a method for measuring the activity of an enzyme - especially the activity of an enzyme belonging to the class of Sirtuins - whereby the enzyme catalyzes an enzymatic reaction in which O-acetyl-ADP-ribose is formed, or ADP-ribose or O-acetyl-ADP-ribose is used as a substrate by the enzyme.

**Figure 3**

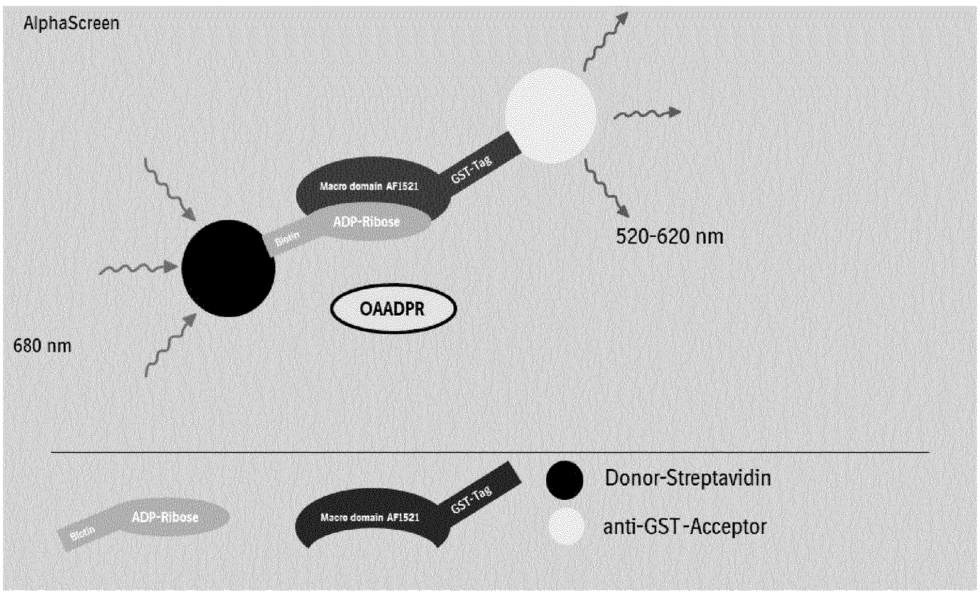


EP 3 076 175 A1

## Description

Field of the invention

The present invention provides a method for measuring the activity of an enzyme - especially the activity of an enzyme belonging to the class of Sirtuins - whereby the enzyme catalyzes an enzymatic reaction in which O-acetyl-ADP-ribose is formed, or ADP-ribose or O-acetyl-ADP-ribose is used as a substrate by the enzyme. Moreover, the present invention also provides a method for identifying potential modulators (e.g. agonist, antagonist) for said enzymes. In a last aspect, also provided is a kit for measuring the activity of said enzyme(s).

Background of the invention

Acetylation and deacetylation of proteins is an important posttranslational modification within cell biology, influencing the functionality of those proteins involved. Acetyl-tranferases are responsible for the acetylation step whereas protein deacetylases are accountable for removing the acetylation. Within mammalians one distinguishes four groups of protein deacetylases. Class 3 deacetylases are composed by the Sirtuin family. Sirtuins are an evolutionarily conserved family of nicotinamide adenine dinucleotide ($NAD^+$) dependent protein-lysin-deacetylases. Currently there are 7 family members known in the human genome. Over the last years there was an increasing interest for these types of enzymes observed, since Sirtuins play a critical role in diverse biological processes. These processes are e.g. regulation of gene expression, control of metabolic processes, apoptosis and cell survival, DNA repair, development, neuroprotection and inflammation. In other words, they control different functions in metabolic processes, stress-response and lifespan and are therefore part of central regulation mechanisms. They can be distinguished from each other by their subcellular localization and substrate specificity. The pharmacological modulation of human Sirtuin activity is considered as an interesting and attractive therapeutic approach for the treatment of lifespan and metabolic diseases, inflammation and immune defense diseases, cardiovascular diseases as well as neuromodulation and cancer.

Different SIRT isoforms perform the following reaction:

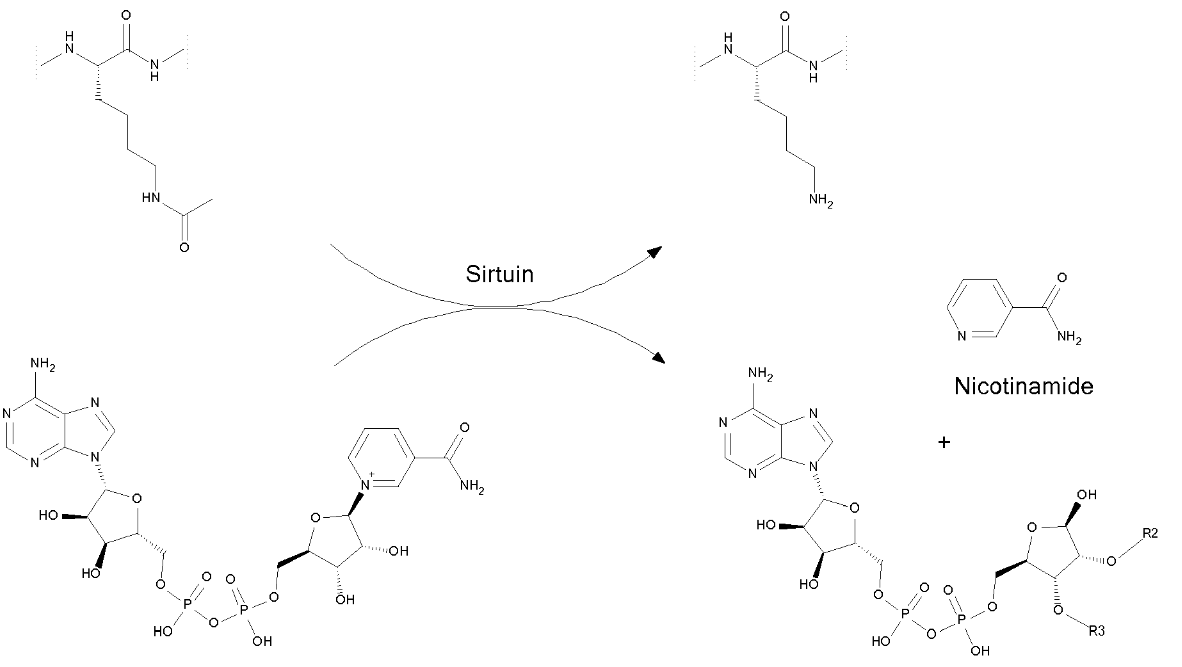


$NAD^+$

2'-O-acetyl-ADP-ribose (R2=acetyl, R3=H)
3'-O-acetyl-ADP-ribose (R2=H, R3=acetyl)

The identification of SIRT modulators e.g. agonists and antagonists in a high throughput screening-compatible format remains difficult. There are different proceedings to reach the goal, but they all have advantages and disadvantages as well as limitations *(*Expert Opin. Drug Discov., (2014), 9(2): 183-199).

Many technologies miss sensitivity especially in miniaturized assay formats, they are prone for interferences during compound testing (side reactions, interference with the read-out technology) or need several different enzymatic reactions to obtain sensitivity *(*Analytical Biochemistry, (2004), 332: 90-99; Journal of Biomolecular Screening, (2011),

16(10): 1227-1235; Anal. Biochem., (2009), 394:101-109; Analytical Biochemistry, (2009), 395: 205-210; Analytical Biochemistry, (2008), 378: 53-59; J. Biol. Chem., (2005), 280: 17187-17195; J. Biol. Chem., (2005), 280: 17038-17045; Anal. Biochem., (2009), 395: 205-210). Sometimes only the usage of radioactively labeled substrates might be successful in terms of setting up sensitive test formats.

The different SIRT enzymes in combination with their different substrate specificities require always a new combination of enzyme and substrate. In consequence of this, working with technologies which require e.g. the modification of a substrate, the reagents always have to be freshly produced.

Currently available technologies focus on the usage of antibodies for the detection of deacetylated lysine residues or on e.g. fluorophore-labeled peptides (acetylated proteins/peptides, deacetylated protein/peptides).

Detecting the consumption of e.g. a generic substrate or detecting the generation of SIRT reaction products ($NAD^+$, 2'/3'-O-acetyl-ADP-ribose) remains difficult since sensitivity is lacking or the usage of several enzymatic reactions is necessary or the technology is not high throughput-compatible *(*Anal. Biochem., (2009), 395: 205-210; Anal. Biochem., (2008), 378: 53-59).

The usage of several enzymatic reactions is not advantageous in the process of compound testing not advantageous since the establishment of counter screen assays to filter out those compounds which do modulate the other enzymes besides SIRT is time consuming and cost intensive.

Newest SIRT activity assays are based on the usage of mass spectrometry-(MS)-systems and focus on the detection of e.g. 2'/3',-O-acetyl-ADP-ribose. They allow the detection of a generic reaction product in a sensitive way. Nevertheless, constraints remain like the need for the enzymatic reaction to be stopped, the need for an internal standard, the samples to be desalted and the availability of expensive MS devices. Exceedingly critical is the fact, that those systems do not allow high throughput-compatible assay runs, meaning e.g. the test of >500.000 compounds in a reasonable period of time in combination with a manageable cost factor (Anal Biochem., (2008), 15, 383,29: 174-179; Journal of Biomolecular Screening, (2011), 16(10): 1217-1226).

<u>Summary of the invention</u>

The present invention was made in view of the difficulties of the prior art to provide a high-throughput-screening (HTS) compatible mix and measure assay format for measuring the activity of enzymes catalyzing an enzymatic reaction, in which either O-acetyl-ADP-ribose is formed, or ADP-ribose or O-acetyl-ADP-ribose is used as a substrate by the enzyme.

The inventors have surprisingly found that by employing (i) a macro-domain protein comprising an acceptor complex, and (ii) ADP-ribose or O-acetyl-ADP-ribose comprising a donor complex, or vice versa, the activity of said enzymes can be measured.

The macro-domain protein belongs to an evolutionarily conserved protein class of Histone binding proteins capable of binding ADP-ribose or O-acetyl-ADP-ribose. In the inventive method the activity of enzymes catalyzing an enzymatic reaction, in which either O-acetyl-ADP-ribose is formed, or ADP-ribose or O-acetyl-ADP-ribose is used as a substrate by the enzyme, is measured by incubating the enzyme and its reaction product with a macro-domain protein and a ADP-ribose or a O-acetyl-ADP-ribose, whereby both the ADP-ribose or O-acetyl-ADP-ribose and the reaction product O-acetyl-ADP-ribose will compete for binding to the macro-domain protein.

The acceptor complex and the donor complex are either already bound to their respective partners, i.e. to the macro-domain protein, or to the ADP-ribose or O-acetyl-ADP-ribose, or vice versa, or can be bound to them in a subsequent step. Thus, when the macro-domain protein comprising the acceptor complex binds to ADP-ribose or O-acetyl-ADP-ribose comprising the donor complex, the donor complex and the acceptor complex are brought into close proximity to each other. The donor complex can now be excited and due to the close proximity the donor complex activates the acceptor complex and a signal is generated.

The invention described above can be employed in general for the measurement of the activity of enzymes, whereby the enzyme catalyzes an enzymatic reaction in which O-acetyl-ADP-ribose is formed. The present invention is particularly useful for measuring the activity of enzymes belonging to the class of Sirtuins.

The general principle of the inventive method also allows monitoring the consumption of ADP-ribose or O-acetyl-ADP-ribose. Therefore, the fundamental principal of the above described invention can be employed in general for the measurement of the activity of enzymes, whereby the enzyme catalyzes an enzymatic reaction in which ADP-ribose or O-acetyl-ADP-ribose is used as a substrate. The present invention is therefore particularly useful for measuring the activity of enzymes belonging to the class of Nudix hydrolases.

The inventive method is especially advantageous because the enzymes and substrates remain unchanged and therefore the actual enzyme reaction is not influenced by the method of measurement. This way, any disturbing artefacts are ruled out and the reaction can be set up in an optimal way.

Furthermore, the present approach allows the free combination of enzymes and their respective substrates. There is no need to design and produce new substrates or enzymes for the different enzyme reactions. Moreover, a

direct detection of the reaction product O-acetyl-ADP-ribose is possible allowing for a quantification of enzyme activity without loss.

The assay set-up is also useful to identify potential modulators (e.g. agonist, antagonist) for said enzymes. If the enzyme catalyzes a reaction in which O-acetyl-ADP-ribose is formed, in such an assay a reduction or the absence of the signal generated in the presence of a modulator is indicative for the agonistic properties of the modulator and an increase of the signal in the presence of the modulator is indicative for the antagonistic properties of the modulator. On the other hand, if the enzyme catalyzes a reaction in which ADP-ribose or O-acetyl-ADP-ribose is used as a substrate, in such an assay a reduction or the absence of the signal generated in the presence of a modulator is indicative for the antagonistic properties of the modulator and an increase of the signal in the presence of the modulator is indicative for the agonistic properties of the modulator.

As will be shown in the examples below, the present invention has sensitivity comparable to systems based on utilizing mass spectrometry. However, the present invention allows for testing ~100.000 compounds within 24h and at the same time realizes savings in costs.

Therefore, the present method can be utilized in a high-through-put setting. Furthermore, the present method eliminates the need for radioactively labeled substrates and can be setup as non-radioactive assay.

The present method is also usable in a mix-and-measure format, hence, eliminating the need for washing, separation, desalting and/or filtration steps.

In a last aspect, also provided is a kit for measuring the activity of an enzyme, whereby the enzyme catalyzes an enzymatic reaction in which O-acetyl-ADP-ribose is formed or in which ADP-ribose or O-acetyl-ADP-ribose is used as a substrate, comprising a macro-domain protein, an ADP-ribose or O-acetyl-ADP-ribose, and an acceptor complex and a donor complex.

Brief description of the drawings

Fig.1: A titration curve with 2'/3'-O-acetyl-ADP-ribose is shown. The 2'/3'-O-acetyl-ADP-ribose concentration is plotted on the X-axis and the strength of the luminescence signal is plotted on the Y-axis. In this titration reaction 2'/3'-O-acetyl-ADP-ribose competes with biotinylated ADP-ribose for binding to the GST-tagged macro-domain protein. With raising 2'/3'-O-acetyl-ADP-ribose levels the luminescence signal decreases.

Fig.2: A titration curve with ADP-ribose is shown. The ADP-ribose concentration is plotted on the X-axis and the strength of the luminescence signal is plotted on the Y-axis. In this titration reaction ADP-ribose competes with biotinylated ADP-ribose for binding to the GST-tagged macro-domain protein. With raising ADP-ribose levels the luminescence signal decreases. The dotted lines indicate the amount of 2'/3'-O-acetyl-ADP-ribose generated by using 1x and 10x SIRT enzyme concentration.

Fig.3: The basic assay principle is illustrated in this specific embodiment. A biotinylated ADP-ribose, a GST-tagged macro-domain protein, a streptavidin coated donor-bead (donor complex) and an anti-GST acceptor-bead (acceptor complex) is utilized. The streptavidin coated donor-bead binds to the biotinylated ADP-ribose and the anti-GST acceptor-bead binds to the GST-tagged macro-domain protein. As long as the biotinylated ADP-ribose is bound to the GST-tagged macro-domain protein, the donor-bead is in close proximity to the acceptor-bead and excitation of the donor-bead generates a signal according to the AlphaScreen principle. 2'/3'-O-acetyl-ADP-ribose (OAADPR) competes with the biotinylated ADP-ribose for binding to the GST-tagged macro-domain protein and the strength of the signal decreases with raising levels of OAADPR.

Fig.4: The inhibition of 10x SIRT with 10 μM EX00001253 is shown. The ADP-ribose concentration is plotted on the X-axis and the strength of the luminescence signal is plotted on the Y-axis. Dotted lines indicate the amount of 2'/3'-O-acetyl-ADP-ribose generated by 10x SIRT reaction and reduction of amount of 2'/3'-O-acetyl-ADP-ribose generated by 10x SIRT inhibited with 10 μM EX00001253.

Fig.5: The inhibition of 10x SIRT with 10 μM Suramin is shown. The ADP-ribose concentration is plotted on the X-axis and the strength of the luminescence signal is plotted on the Y-axis. Dotted lines indicate the amount of 2'/3'-O-acetyl-ADP-ribose generated by 10x SIRT reaction and reduction of amount of 2'/3'-O-acetyl-ADP-ribose generated by 10x SIRT inhibited with 10 μM Suramin.

Fig.6: The MS-Spectrum of GST-tagged AF1521 is shown.

Fig.7: The binding of biotinylated ADP-ribose to GST-tagged AF1521 measured by isothermal calorimetry (ITC) is shown. GST-tagged AF1521 (50 μM) was titrated with biotinylated ADP-ribose (500 μM). Top and lower panels correspond to the titration kinetics and the binding isotherm, respectively. Regression analysis with the software ORIGIN 7 is indicated with a red trace (bottom panel). In the upper panel the X-axis shows the time in minutes and the Y-axis shows the experimental heat change in μcal/sec resulting from injection. In the lower panel the X-axis shows the molar ratio of AF1521 and biotinylated ADP-ribose and the Y-axis shows the integrated heat change in kcal per mol injectant.

Fig.8: The binding of acetylated ADP-ribose to GST-tagged AF1521 measured by isothermal calorimetry (ITC) is shown. GST-tagged AF1521 (50 μM) was titrated with acetylated ADP-ribose (500 μM). Top and lower panels correspond to the titration kinetics and the binding isotherm, respectively. Regression analysis with the software ORIGIN 7 is indicated with a red trace (bottom panel). In the upper panel the X-axis shows the time in minutes and the Y-axis shows the experimental heat change in μcal/sec resulting from injection. In the lower panel the X-axis shows the molar ratio of AF1521 and acetylated ADP-ribose and the Y-axis shows the integrated heat change in kcal per mol injectant.

Detailed description of the invention

The present invention is directed to a method for measuring the activity of an enzyme, whereby the enzyme catalyzes an enzymatic reaction in which either (ia) O-acetyl-ADP-ribose is formed, or (ib) ADP-ribose or O-acetyl-ADP-ribose is used as a substrate by the enzyme, the method comprising the steps of:

(ia) incubating the enzyme with its substrate(s) such that O-acetyl-ADP-ribose is formed, or

(ib) incubating the enzyme with ADP-ribose or O-acetyl-ADP-ribose, which is then used as substrate by the enzyme, and

(ii) adding to step (ia) or step (ib) a macro-domain protein and a ADP-ribose or O-acetyl-ADP-ribose, whereby the macro-domain protein comprises an acceptor complex or is bound to an acceptor complex in a further step and the ADP-ribose or O-acetyl-ADP-ribose comprises a donor complex or is bound to a donor complex in a further step, or vice versa, and

whereby the ADP-ribose or O-acetyl-ADP-ribose competes with the ADP-ribose or O-acetyl-ADP-ribose of step (ia) or step (ib) for binding to the macro-domain protein, and whereby the donor complex comprises a molecule that can be excited and the acceptor complex comprises a molecule that can be stimulated by the excited donor complex, if both are in close proximity to each other, and

(iii) exciting the mixture of step (ii), and

(iv) measuring the signal arising upon excitation.

The method can be applied to measure the activity of any enzyme capable of catalyzing a reaction in which O-acetyl-ADP-ribose is formed. Such enzymes are known in the art and comprise amongst others $NAD^+$-dependent protein deacetylases. In a preferred embodiment the $NAD^+$-dependent protein deacetylase belongs to the class of Sirtuins. Thus, in a preferred embodiment the activity of Sirtuins is measured. In yet another embodiment the substrate comprises an acetylated protein and $NAD^+$.

The method can also be applied to measure the activity of enzymes that use ADP-ribose or O-acetyl-ADP-ribose as a substrate. Such enzymes are known in the art and comprise amongst others enzymes that belong to the family of Nudix hydrolases or to the family of Pyrophosphatases.

The enzymes capable of catalyzing a reaction in which O-acetyl-ADP-ribose is formed usually produce 2'-O-acetyl-ADP-ribose or 3'-O-acetyl-ADP-ribose or a mixture thereof. The method can reliably detect each of said reaction products and does not distinguish between them. Hence, the term O-acetyl-ADP-ribose refers to either 2'-O-acetyl-ADP-ribose or 3'-O-acetyl-ADP-ribose, and/or the mixture 2'/3'-O-acetyl-ADP-ribose.

The macro-domain protein belongs to a highly evolutionarily conserved domain with homologues in viruses, archaea, bacteria, invertebrates, amphibians, mammals, and plants. Mostly common to all macro-domain proteins is usually an approximately 130-190 amino acid conserved domain which allows them to bind the various forms of ADP-ribose. The macro-domain can recognize ADP-ribose or poly-ADP-ribose. The 3D structure of the macro-domain has a mixed alpha/beta fold of a mixed beta sheet sandwiched between four helices.

Macro-domain proteins that can be used in the present invention comprise human H2AY_human, MacroH2A.2, LRP16/MD01, C6orf130, C20orf133/MD02, GDAP2/MDO3, CHDIL/ALC1, PARP-9(BAL1), PARP-15(BAL3), PARP-14(BAL2/CoaSt6).

Macro-domain proteins from other organisms can also be used and comprise E.c.C4ZRY6, X.Q6GP69, D.Q961L8, A.t.Q9M041, O.s.Is03g0336500, Y1521_ARCFU, V.SFV(nsp3), V.SARS(msp3), V.HEV ORF.

In an embodiment the macro-domain protein is used from a virus, archaea, bacteria, invertebrate, amphibian, plant and/or mammal. Preferably, human or archae macro-domain proteins are used.

In a preferred embodiment the macro-domain protein comprises a sequence as set forth in SEQ ID NO:1 or a sequence that is at least 50%, 55%, 60%, 65%, 70%, 75% ,80%, 85%, 90%, 95%, or 99% identical to that sequence.

In a preferred embodiment the macro-domain protein is AF1521 (Y1521_ARCFU or 028751) with the sequence:

MEVLFEAKVGDITLKLAQGDITQYPAKAIVNAANKRLEHGGGVAYAIAKACAGDAGLYTEISKKAMREQF
GRDYIDHGEVVVTPAMNLEERGIKYVFHTVGPICSGMWSEELKEKLYKAFLGPLEKAEEMGVESIAFPAVS
AGIYGCDLEKVVETFLEAVKNFKGSAVKEVALVIYDRKSAEVALKVFERSL (SEQ ID NO:1)

In another preferred embodiment the macro-domain protein comprises a sequence as set forth in SEQ ID NO:2 or a sequence that is at least 50%, 55%, 60%, 65%, 70%, 75% ,80%, 85%, 90%, 95% or 99% identical to that sequence.

In yet another embodiment the macro-domain protein is human H2AY (mH2A1 or 075367) with the sequence:

MSSRGGKKKSTKTSRSAKAGVIFPVGRMLRYIKKGHPKYRIGVGAPVYMAAVLEYLTAEILELAGNAARD
NKKGRVTPRHILLAVANDEELNQLLKGVTIASGGVLPNIHPELLAKKRGSKGKLEAIITPPPAKKAKSPSQKK
PVSKKAGGKKGARKSKKKQGEVSKAASADSTTEGTPADGFTVLSTKSLFLGQKLNLIHSEISNLAGFEVEAII
NPTNADIDLKDDLGNTLEKKGGKEFVEAVLELRKKNGPLEVAGAAVSAGHGLPAKFVIHCNSPVWGADK
CEELLEKTVKNCLALADDKKLKSIAFPSIGSGRNGFPKQTAAQLILKAISSYFVSTMSSSIKTVYFVLFDSESIGI
YVQEMAKLDAN (SEQ ID NO:2)

In another embodiment the macro-domain protein comprises the amino acids 184-370 of the sequence as set forth in SEQ ID NO:2 or a sequence that is at least 50%, 55%, 60%, 65%, 70%, 75% ,80%, 85%, 90%, 95% or 99% identical to that 184-370 amino acids.

Sequence identity can be determined by methods known in the art such as BLAST.

The "donor complex" is comprised of at least one molecule that can be excited and subsequently stimulates the acceptor complex, if both are in close proximity to each other. Usually, this molecule is a dye that can be stimulated with light of specific wavelengths. The stimulated molecule then itself emits light of a certain wavelength or generates other stimuli that are able to activate the acceptor complex e.g. reactive oxygen species.

The "acceptor complex" is comprised of at least one molecule that can be stimulated by the excited donor complex, if both are in close proximity to each other. Stimulation of the acceptor complex by the donor complex leads to the generation of a signal that can be measured. Usually, this is light emitting at a certain wavelength.

Excitation of the donor complex can be achieved e.g. with a laser emitting at a certain wavelength. However, the source and exact nature of excitation depends mainly on the actual donor and acceptor complex used. It is within the knowledge of the skilled artisan to choose the appropriate excitation source. Similarly, measurement of the signal arising upon excitation depends also on the aforementioned parameters but is usually measured with a photometric device.

The term "close proximity to each other" refers preferably to a distance of 500nm or 400nm or 300nm or 250nm or 200nm or 150nm or 100nm or 50nm.

Thus, the common nature of all these systems is the formation of a proximity detection system by the acceptor complex and the donor complex. The proximity detection system can be based on different principles such as fluorescence resonance energy transfer (FRET), scintillation, luminescence, radioactivity, bioluminescence, chemoluminescence or electrochemoluminescence.

In a preferred embodiment the acceptor complex and the donor complex comprises a bead. In yet another embodiment the donor bead is conjugated with streptavidin and the acceptor bead is conjugated with anti-GST.

In another embodiment the donor complex and the acceptor complex form an amplified luminescent proximity homogeneous assay. Such a system is well known in the art and e.g. is known under the trademark AlphaScreen™ (PerkinElmer).

It is to be understood that the exact nature of the proximity detection system is not crucial for the present method. The proximity detection system serves as a tool to visualize the activity of the enzymes in the inventive method.

In the inventive method a macro-domain protein and either ADP-ribose or O-acetyl-ADP-ribose are added in step (ii), whereby the macro-domain protein comprises an acceptor complex or is bound to an acceptor complex in a further step and the ADP-ribose or O-acetyl-ADP-ribose comprises a donor complex or is bound to a donor complex in a further step, or vice versa.

Thus, it is possible to add a macro-domain protein and either ADP-ribose or O-acetyl-ADP-ribose that already comprises, i.e. is already bound/conjugated to an acceptor complex and a donor complex. The acceptor complex can be bound/conjugated to the macro-domain protein and the donor complex to the ADP-ribose or O-acetyl-ADP-ribose, or vice versa, this means that the acceptor complex can also be bound/conjugated to the ADP-ribose or O-acetyl-ADP-ribose and the donor complex to the macro-domain protein.

The acceptor and donor complex can be either directly bound/conjugated to the macro-domain protein and the ADP-ribose or O-acetyl-ADP-ribose, or they can be bound/conjugated "indirectly" with a tag. The term "tag" or "tagged" as used herein refers to a molecule which is capable of being bound, attached and/or conjugated to a macro-domain protein or to ADP-ribose or O-acetyl-ADP-ribose and which thereafter can be detected specifically by another molecule.

The term "specific for" refers to a pair of molecules which are naturally derived or synthetically produced, wherein one of the pair of molecules, has certain structural features that are complementary with a particular structure or organization of the other molecule, so that the molecules have the property of binding specifically to each other. These are for example antibody-antigen, biotin-streptavidin, receptor-ligand, enzyme-substrate bindings.

As outlined above, the macro-domain protein and the ADP-ribose or O-acetyl-ADP-ribose can already comprise the acceptor and donor complex. However, it is also possible to add in step (ii) a macro-domain protein and either ADP-ribose or O-acetyl-ADP-ribose that is not already bound/conjugated to an acceptor or donor complex. In such a setting the macro-domain protein and the ADP-ribose or O-acetyl-ADP-ribose is added first and then in a further step the acceptor complex and the donor complex are added to the mixture. To achieve a specific binding, the usage of a tag is most preferred, i.e. the macro-domain protein and the ADP-ribose or O-acetyl-ADP-ribose is tagged and the corresponding partner of the tag is bound/conjugated to the acceptor complex or the donor complex, respectively. In one setting the tag is a hapten molecule (e.g., biotin), which can be bound by streptavidin. Also, in another setting the tag is a GST-tag (Glutathione S-transferase), which subsequently can be bound by anti-GST. In a preferred embodiment the macro-domain protein is tagged with GST and the ADP-ribose or O-acetyl-ADP-ribose is tagged with biotin or vice versa.

It is to be understood that the tag only serves the purpose to provide a specific linkage to the donor or the acceptor complex respectively. As such, the specific nature of the tag is not crucial as long as the tag does not interfere with the binding of the tagged macro-domain protein or the tagged ADP-ribose or O-acetyl-ADP-ribose to their respective substrates.

The enzyme catalyzing the enzymatic reaction in which either (ia) O-acetyl-ADP-ribose is formed, or (ib) ADP-ribose or O-acetyl-ADP-ribose is used as a substrate, can be either recombinantly expressed or can be purified from a natural source. Also, whole cell lysates can be measured for activity of the enzyme. Such whole cell lysates can be either purified beforehand or can be used without purification. The activity of said enzymes can also be measured in whole cell cultures. In such a setup the step (i) of the method is omitted, as this reaction has already occurred in the cells. Instead, the cells may be lysed and/or fixed before proceeding directly with step (ii) of the method.

In a preferred embodiment the method is directed to measuring the activity of a $NAD^+$-dependent protein deacetylase, whereby the $NAD^+$-dependent protein deacetylase catalyzes an enzymatic reaction in which O-acetyl-ADP-ribose is formed, the method comprising the steps of:

(i) incubating the $NAD^+$-dependent protein deacetylase with its substrate(s) such that O-acetyl-ADP-ribose is formed, and

(ii) adding to step (i) a macro-domain protein and a ADP-ribose or O-acetyl-ADP-ribose, whereby the macro-domain protein comprises an acceptor complex or is bound to an acceptor complex in a further step and the ADP-ribose or O-acetyl-ADP-ribose comprises a donor complex or is bound to a donor complex in a further step, or vice versa, and whereby the ADP-ribose or O-acetyl-ADP-ribose competes with the ADP-ribose or O-acetyl-ADP-ribose of step (i) for binding to the macro-domain protein, and whereby the donor complex comprises a molecule that can be excited and the acceptor complex comprises a molecule that can be stimulated by the excited donor complex, if both are in close proximity to each other, and

(iii) exciting the mixture of step (ii), and

(iv) measuring the signal arising upon excitation.

In yet another embodiment the method is directed to measuring the activity of a Nudix hydrolases, whereby the Nudix hydrolases catalyzes an enzymatic reaction in which ADP-ribose or O-acetyl-ADP-ribose is used as a substrate, the method comprising the steps of:

(i) incubating the Nudix hydrolases with ADP-ribose or O-acetyl-ADP-ribose, which is then used as substrate by the Nudix hydrolases, and

(ii) adding to step (i) a macro-domain protein and a ADP-ribose or O-acetyl-ADP-ribose, whereby the macro-domain protein comprises an acceptor complex or is bound to an acceptor complex in a further step and the ADP-ribose or O-acetyl-ADP-ribose comprises a donor complex or is bound to a donor complex in a further step, or vice versa, and whereby the ADP-ribose or O-acetyl-ADP-ribose competes with the ADP-ribose or O-acetyl-ADP-ribose of step (i) for binding to the macro-domain protein, and whereby the donor complex comprises a molecule that can be excited and the acceptor complex comprises a molecule that can be stimulated by the excited donor complex, if both are in close proximity to each other, and

(iii) exciting the mixture of step (ii), and

(iv) measuring the signal arising upon excitation.

In another embodiment the method is directed to measuring the activity of a Sirtuin, whereby the Sirtuin catalyzes an enzymatic reaction in which O-acetyl-ADP-ribose is formed, the method comprising the steps of:

(i) incubating the Sirtuin with its substrate(s) such that O-acetyl-ADP-ribose is formed, and

(ii) adding to step (i) a macro-domain protein and a ADP-ribose or O-acetyl-ADP-ribose, whereby the macro-domain protein comprises an acceptor complex or is bound to an acceptor complex in a further step and the ADP-ribose or O-acetyl-ADP-ribose comprises a donor complex or is bound to a donor complex in a further step, or vice versa, and whereby the ADP-ribose or O-acetyl-ADP-ribose competes with the ADP-ribose or O-acetyl-ADP-ribose of step (i) for binding to the macro-domain protein, and whereby the donor complex comprises a molecule that can be excited and the acceptor complex comprises a molecule that can be stimulated by the excited donor complex, if both are in close proximity to each other, and

(iii) exciting the mixture of step (ii), and

(iv) measuring the signal arising upon excitation.

In another embodiment the method is directed to measuring the activity of a Sirtuin, whereby the Sirtuin catalyzes an enzymatic reaction in which O-acetyl-ADP-ribose is formed, the method comprising the steps of:

(i) incubating the Sirtuin with an acetylated peptide substrate and $NAD^+$ such that O-acetyl- ADP-ribose is formed, and

(ii) adding to step (i) a macro-domain protein and a ADP-ribose or O-acetyl-ADP-ribose, whereby the macro-domain protein comprises an acceptor complex or is bound to an acceptor complex in a further step and the ADP-ribose or O-acetyl-ADP-ribose comprises a donor complex or is bound to a donor complex in a further step, or vice versa, and whereby the ADP-ribose or O-acetyl-ADP-ribose competes with the ADP-ribose or O-acetyl-ADP-ribose of step (i) for binding to the macro-domain protein, and whereby the donor complex comprises a molecule that can be excited and the acceptor complex comprises a molecule that can be stimulated by the excited donor complex, if both are in close proximity to each other, and

(iii) exciting the mixture of step (ii), and

(iv) measuring the signal arising upon excitation.

In yet another embodiment the method is directed to measuring the activity of a Sirtuin, whereby the Sirtuin catalyzes an enzymatic reaction in which O-acetyl-ADP-ribose is formed, the method comprising the steps of:

(i) incubating the Sirtuin with an acetylated peptide substrate and $NAD^+$ such that O-acetyl-ADP-ribose, is formed, and

(ii) adding to step (i) a tagged macro-domain protein and a tagged ADP-ribose or tagged O-acetyl-ADP-ribose, whereby the tagged macro-domain protein comprises an acceptor complex or is bound to an acceptor complex in a further step and the tagged ADP-ribose or tagged O-acetyl-ADP-ribose comprises a donor complex or is bound to a donor complex in a further step, or vice versa, and
whereby the tagged ADP-ribose or tagged O-acetyl-ADP-ribose competes with the ADP-ribose or O-acetyl-ADP-ribose of step (i) for binding to the macro-domain protein, and whereby the donor complex comprises a molecule that can be excited and the acceptor complex comprises a molecule that can be stimulated by the excited donor complex, if both are in close proximity to each other, and

(iii) exciting the mixture of step (ii), and

(iv) measuring the signal arising upon excitation.

In yet another embodiment the method is directed to measuring the activity of a Sirtuin, whereby the Sirtuin catalyzes an enzymatic reaction in which O-acetyl-ADP-ribose is formed, the method comprising the steps of:

(i) incubating the Sirtuin with an acetylated peptide substrate and $NAD^+$ such that O-acetyl-ADP-ribose, is formed, and

(ii) adding to step (i) a GST-tagged macro-domain protein and a biotin-tagged ADP-ribose or biotin-tagged O-acetyl-ADP-ribose, whereby the GST-tagged macro-domain protein comprises an acceptor complex or is bound to an acceptor complex in a further step and the biotin-tagged ADP-ribose or biotin-tagged O-acetyl-ADP-ribose comprises a donor complex or is bound to a donor complex in a further step, or vice versa, and
whereby the biotin-tagged ADP-ribose or biotin-tagged O-acetyl-ADP-ribose competes with the ADP-ribose or O-acetyl-ADP-ribose of step (i) for binding to the GST-tagged macro-domain protein, and whereby the donor complex comprises a molecule that can be excited and the acceptor complex comprises a molecule that can be stimulated by the excited donor complex, if both are in close proximity to each other, and

(iii) exciting the mixture of step (ii), and

(iv) measuring the signal arising upon excitation.

In another embodiment the method is directed to measuring the activity of a Sirtuin, whereby the Sirtuin catalyzes an enzymatic reaction in which O-acetyl-ADP-ribose is formed, the method comprising the steps of:

(i) incubating the Sirtuin with an acetylated peptide substrate and $NAD^+$ such that O-acetyl-ADP-ribose, is formed, and

(ii) adding to step (i) a GST-tagged macro-domain protein and a biotin-tagged ADP-ribose or biotin-tagged O-acetyl-ADP-ribose,

(iii) adding to step (ii) an anti-GST acceptor bead comprising an acceptor complex, and a streptavidin coated donor bead comprising a donor complex, whereby the anti-GST acceptor bead binds the GST-tagged macro-domain protein and the streptavidin coated donor bead binds the biotin-tagged ADP-ribose,
whereby the biotin-tagged ADP-ribose or biotin-tagged O-acetyl-ADP-ribose competes with the ADP-ribose or O-acetyl-ADP-ribose of step (i) for binding to the GST-tagged macro-domain protein, and whereby the donor complex comprises a molecule that can be excited and the acceptor complex comprises a molecule that can be stimulated by the excited donor complex, if both are in close proximity to each other, and

(iv) exciting the mixture of step (ii), and

(v) measuring the signal arising upon excitation.

In yet another embodiment the method is directed to measuring the activity of a Sirtuin, whereby the Sirtuin catalyzes an enzymatic reaction in which O-acetyl-ADP-ribose is formed, the method comprising the steps of:

(i) incubating a Sirtuin with an acetylated peptide substrate and NAD+ such that O-acetyl-ADP-ribose is formed, and

(ii) adding to the product(s) of step (i) a GST-tagged macro-domain protein and a biotin-tagged ADP-ribose, and adding in a further step an anti-GST acceptor bead and a streptavidin coated donor bead, whereby the anti-GST

acceptor bead binds the GST-tagged macro-domain protein and the streptavidin coated donor bead binds the biotin-tagged ADP-ribose, and

(iii) exciting the mixture of step (ii) with a light source, and

(iv) measuring the signal arising upon excitation

The invention also provides for a method for identifying potential modulators of an enzyme, whereby the enzyme catalyzes an enzymatic reaction in which either (ia) O-acetyl-ADP-ribose is formed, or (ib) ADP-ribose or O-acetyl-ADP-ribose is used as a substrate by the enzyme, the method comprising the steps of:

(ia) performing the steps of (ia)-(iv) of any of the described methods, whereby the potential modulator of the enzyme is added before or during any of steps (ia)-(iv), or

(ib) performing the steps of (ib)-(iv) of any of the described methods, whereby the potential modulator of the enzyme is added before or during any of steps (ib)-(iv), and

(iia) wherein after performing the steps of (ia)-(iv), a reduction or the absence of the signal obtained in the presence of the modulator is indicative for the agonistic properties of the modulator and wherein an increase of the signal in the presence of the modulator is indicative for the antagonistic properties of the modulator, or

(iib) wherein after performing the steps of (ib)-(iv), a reduction or the absence of the signal obtained in the presence of the modulator is indicative for the agonistic properties of the modulator and wherein an increase of the signal in the presence of the modulator is indicative for the antagonistic properties of the modulator.

A modulator or potential modulator is either an inhibitor or an activator of the enzyme reaction.

In another embodiment the method is directed to identifying potential modulators of a Sirtuin, whereby the Sirtuin catalyzes an enzymatic reaction in which O-acetyl-ADP-ribose is formed, the method comprises the steps of:

(i) performing the steps of (ia)-(iv) of any of the described methods, whereby the potential modulator of the enzyme is added before or during any of steps (ia)-(iv), and

(ii) wherein after performing the steps of (ia)-(iv), a reduction or the absence of the signal obtained in the presence of the modulator is indicative for the agonistic properties of the modulator and wherein an increase of the signal in the presence of the modulator is indicative for the antagonistic properties of the modulator.

Using the macro-domain protein and the ADP-ribose or O-acetyl-ADP-ribose in combination with a close proximity detection system, a competition assay can be set up. The macro-domain protein can be bound e.g. to an acceptor-bead comprising an acceptor complex, whereas the ADP-ribose can be bound to a donor-bead comprising a donor complex. If the macro-domain protein binds the ADP-ribose, donor- and acceptor-bead come into close proximity and a signal is generated after excitation of the acceptor complex. In those cases were O-acetyl-ADP-ribose is produced by the enzyme reaction such produced O-acetyl-ADP-ribose will compete with the ADP-ribose for the binding site of the macro-domain protein and therefore the signal generated by the close proximity system will decrease.

Hence, in general, a reduction or the absence of the signal obtained in the presence of the modulator is indicative for the agonistic properties of the modulator and wherein an increase of the signal in the presence of the modulator is indicative for the antagonistic properties of the modulator. However, it is also possible to employ an ADP-standard curve and thereby quantify the amount of produced O-acetyl-ADP-ribose.

The present invention also provides for a kit for measuring the activity of an enzyme, whereby the enzyme catalyzes an enzymatic reaction in which either (ia) O-acetyl-ADP-ribose is formed, or (ib) ADP-ribose or O-acetyl-ADP-ribose is used as a substrate by the enzyme, comprising

(i) a macro-domain protein and a ADP-ribose or O-acetyl-ADP-ribose, and

(ii) an acceptor and a donor complex, and

(iii) (optionally) substrate(s) for the enzyme.

In a preferred embodiment a kit is provided for measuring the activity of a Sirtuin, whereby the Sirtuin catalyzes

an enzymatic reaction in which O-acetyl-ADP-ribose is formed, comprising

(i) a macro-domain protein and a ADP-ribose or O-acetyl-ADP-ribose, and

(ii) an acceptor and a donor complex, and

(iii) (optionally) substrate(s) for the enzyme.

Further preferred is a kit for measuring the activity of a Sirtuin, whereby the Sirtuin catalyses an enzymatic reaction in O-acetyl-ADP-ribose is formed, comprising

(i) a GST-tagged macro-domain protein and a biotin-tagged ADP-ribose,

(ii) an anti-GST acceptor bead and a streptavidin coated donor bead, and

(iii) (optionally) an acetylated peptide substrate and NAD+.

In yet another embodiment the kit is directed to the measurement of activity of Nudix hydrolases.

The present invention also provides for the use of a macro-domain protein, and either ADP-ribose or O-acetyl-ADP-ribose, and an acceptor complex and a donor complex to measure the activity of an enzyme, whereby the enzyme catalyzes an enzymatic reaction in which either O-acetyl-ADP-ribose is formed or ADP-ribose or O-acetyl-ADP-ribose is used as a substrate by the enzyme.

## Examples

### Example 1:

### Cloning, expression and purification of macro-domain proteins

The gene corresponding to amino acid residues 162-369 from mH2A1.1 (H2AY_human, Uniprot ID 075367) was produced by gene synthesis (optimized for E. coli expression) with an N-terminal His-tag followed by a TEV protease site or with a N-terminal StrepII Tag followed by a TEV protease site and cloned into pET24a (Novagen) with NdeI and EcoRI. For the N-terminal GST fusion of H2A1.1 the gene for residues 162-369 was synthesized and cloned into pGEX 4T-1 (Pharmacia) with BamH1 and XhoI.

The gene for the macro-domain AF1521 from Archaeoglobus fulgidus (Y1521_ARCFU, Uniprot ID 028751) amino acid residues 1-192 was produced by gene synthesis with a N-terminal His-tag fusion followed by a TEV protease site and cloned into pET24a with BamH1 and XhoI. For the N-terminal GST fusion of AF1521 the gene for residues 1-192 was synthesized and cloned into pGEX 4T-1 (Pharmacia) with BamH1 and XhoI. For the N-terminal Strep-tag fusion of AF1521 the gene for residues 1-192 was synthesized with an N-terminal Strep-tag followed by a TEV protease site and cloned into pET24a with NdeI and EcoRI.

The plasmid for the GST fusion of AF1521 protein was transformed in BL21 Star (DE3) One Shot BL21 Tuner (DE3) (Novagen). Cells were grown in LB containing 100 μM Ampicillin at 37°C to mid log phase and protein expression was induced with 1 mM IPTG. The temperature was then reduced and cells were grown for further 16 h at 20°C. Cells were harvested, resuspended in lysis buffer (PBS with 10% glycerol, 1 mM $MgCl_2$, 2 mM TCEP, protease inhibitors (Complete EDTA-free, Roche Diagnostics), pH 7.4 and lysed by Constant Cell Disruption System.

The fusion protein was purified using GT Sepharose 4B (GE Healthcare) affinity and eluted with PBS buffer containing 20 mM GSH, 2 mM TCEP and 10% glycerol, pH 8.0. AF1521 was purified by gel filtration using Superdex 200 HR column (GE Healthcare) equilibrated in 50 mM Tris pH 8.0, 300 mM NaCl, 0.1 mM EDTA and 1 mM DTT. The protein was identified by MS spectroscopy with the N-terminal Methione removed (cf. Fig.6).

### Protein sequences

AF1521 N-His cleaved:

GMEVLFEAKVGDITLKLAQGDITQYPAKAIVNAANKRLEHGGGVAYAIAKACAGDAGLYTEISKKAMREQ FGRDYIDHGEVVVTPAMNLEERGIKYVFHTVGPICSGMWSEELKEKLYKAFLGPLEKAEEMGVESIAFPAV SAGIYGCDLEKVVETFLEAVKNFKGSAVKEVALVIYDRKSAEVALKVFERSL (SEQ ID NO: 3)

AF1521 N-His:

MASMTGGQQMGRGSMGSHHHHHHSSGENLYFQGMEVLFEAKVGDITLKLAQGDITQYPAKAIVNAA NKRLEHGGGVAYAIAKACAGDAGLYTEISKKAMREQFGRDYIDHGEVVVTPAMNLEERGIKYVFHTVGPI CSGMWSEELKEKLYKAFLGPLEKAEEMGVESIAFPAVSAGIYGCDLEKVVETFLEAVKNFKGSAVKEVALVI YDRKSAEVALKVFERSL (SEQ ID NO: 4)

AF1521 N-GST:

MLGGCPKERAEISMLEGAVLDIRYGVSRIAYSKDFETLKVDFLSKLPEMLKMFEDRLCHKTYLNGDHVTHP DFMLYDALDVVLYMDPMCLDAFPKLVCFKKRIEAIPQIDKYLKSSKYIAWPLQGWQATFGGGDHPPKSD LVPRGSMEVLFEAKVGDITLKLAQGDITQYPAKAIVNAANKRLEHGGGVAYAIAKACAGDAGLYTEISKKA MREQFGRDYIDHGEVVVTPAMNLEERGIKYVFHTVGPICSGMWSEELKEKLYKAFLGPLEKAEEMGVESI AFPAVSAGIYGCDLEKVVETFLEAVKNFKGSAVKEVALVIYDRKSAEVALKVFERSL (SEQ ID NO: 5)

AF1521 N-Strep:

MWSHPQFEKGGGSGGGSGGSAWSHPQFEKGSENLYFQMEVLFEAKVGDITLKLAQGDITQYPAKAIVN AANKRLEHGGGVAYAIAKACAGDAGLYTEISKKAMREQFGRDYIDHGEVVVTPAMNLEERGIKYVFHTV GPICSGMWSEELKEKLYKAFLGPLEKAEEMGVESIAFPAVSAGIYGCDLEKVVETFLEAVKNFKGSAVKEV ALVIYDRKSAEVALKVFERSL (SEQ ID NO: 6)

H2AY N-HisTEV:

MGSHHHHHHSSGENLYFQGFTVLSTKSLFLGQKLNLIHSEISNLAGFEVEAIINPTNADIDLKDDLGNTLEK KGGKEFVEAVLELRKKNGPLEVAGAAVSAGHGLPAKFVIHCNSPVWGADKCEELLEKTVKNCLALADDKK LKSIAFPSIGSGRNGFPKQTAAQLILKAISSYFVSTMSSSIKTVYFVLFDSESIGIYVQEMAKLDAN (SEQ ID NO: 7)

H2AY N-Strep:

MWSHPQFEKGGGSGGGSGGSAWSHPQFEKGSENLYFQGFTVLSTKSLFLGQKLNLIHSEISNLAGFEVE AIINPTNADIDLKDDLGNTLEKKGGKEFVEAVLELRKKNGPLEVAGAAVSAGHGLPAKFVIHCNSPVWGA DKCEELLEKTVKNCLALADDKKLKSIAFPSIGSGRNGFPKQTAAQLILKAISSYFVSTMSSSIKTVYFVLFDSES IGIYVQEMAKLDAN (SEQ ID NO: 8)

H2AY N-GST:

MLGGCPKERAEISMLEGAVLDIRYGVSRIAYSKDFETLKVDFLSKLPEMLKMFEDRLCHKTYLNGDHVTHP
DFMLYDALDVVLYMDPMCLDAFPKLVCFKKRIEAIPQIDKYLKSSKYIAWPLQGWQATFGGGDHPPKSD
LVPRGSGFTVLSTKSLFLGQKLNLIHSEISNLAGFEVEAIINPTNADIDLKDDLGNTLEKKGGKEFVEAVLELR
KKNGPLEVAGAAVSAGHGLPAKFVIHCNSPVWGADKCEELLEKTVKNCLALADDKKLKSIAFPSIGSGRN
GFPKQTAAQLILKAISSYFVSTMSSSIKTVYFVLFDSESIGIYVQEMAKLDAN (SEQ ID NO: 9)

**Example 2:**

**Biotinylation of ADP-ribose**

For the synthesis of a biotinylated ADP-ribose ligand a similar strategy was followed as described in J. Am. Chem. Soc. 132, 2010:15878, where an oxime function is utilized to couple the ADP-ribose to a biotin reagent.

ADP-ribose was biotinylated according to the following reaction scheme:

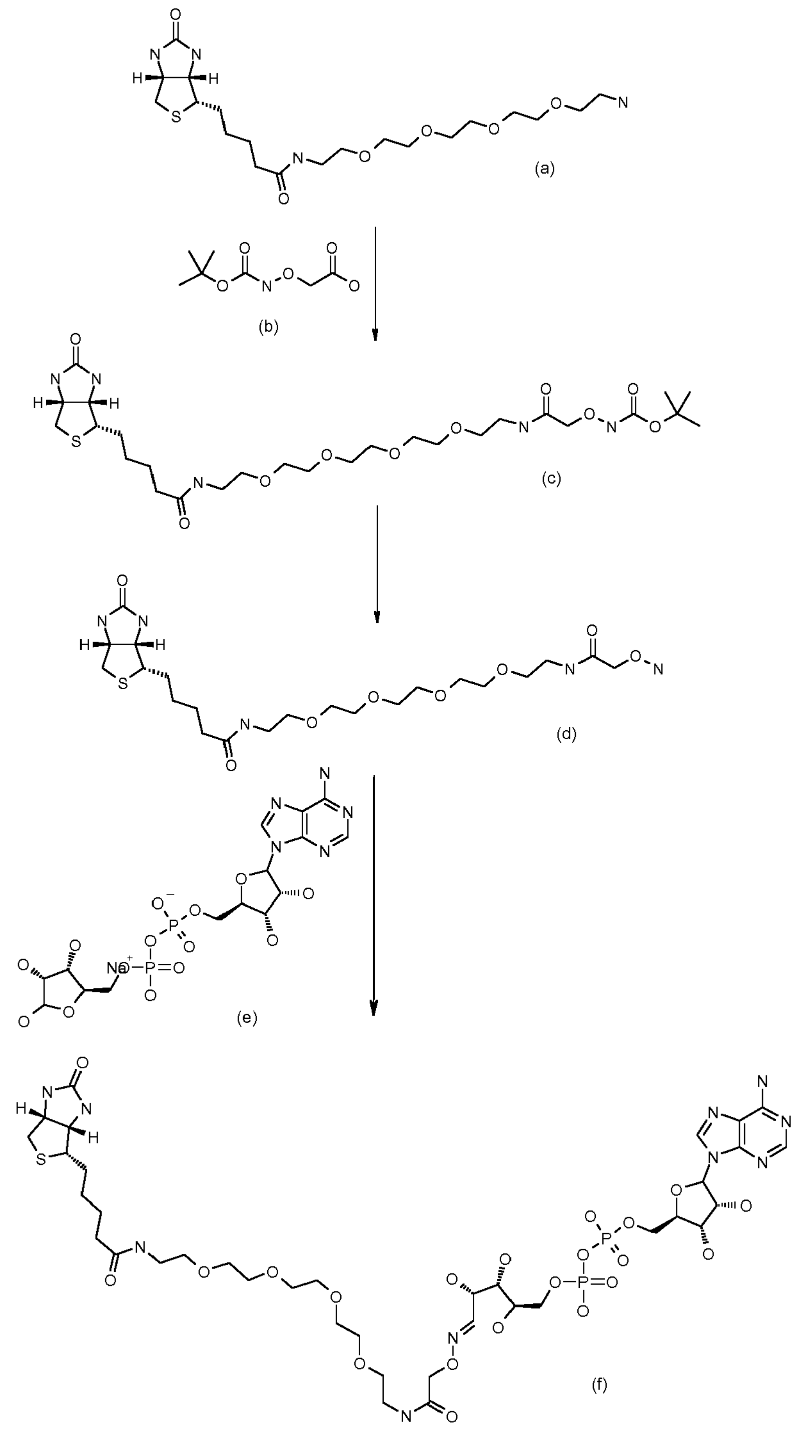

(a)

(b)

(c)

(d)

(e)

(f)

**biotinylated ADP-ribose (MW. 1076.96 g/Mol)**

The following compounds were used:

Compound (a): available by Synchem-Inc; biotin-PEG4-$NH_2$; SC50087

Compound (b): available by Sigma-Aldrich; (BOC-aminooxy) acetic acid; 15035-5g

Compound (e): available by Sigma-Aldrich; adenosine-5'-diphosphoribose-sodium salt, A0752-100mg

The following chromatography methods were used:

**Preparative HPLC Method (1):**

HPLC: Gilson, GX-281 Preparative HPLC System

Column: Waters XBridge C18, 10 µm, even packed Modcol System, ca. 5 x 25 cm, temperature: room temperature

Solvent: A: demineralized water with 0.2 % $NH_4OH$; B: acetonitrile, HPLC grade

Table 1: Gradients

| **time (min.)** | **A** | **B** | **flow (ml/min)** |
|---|---|---|---|
| 0,00 | 95 | 5 | 150 |
| 0,80 | 95 | 5 | 180 |
| 2,00 | 95 | 5 | 180 |
| 13,00 | 0 | 100 | 180 |
| 16,00 | 0 | 100 | 180 |
| 16,50 | 95 | 5 | 180 |
| 19,00 | 95 | 5 | 180 |

**Analytic HPLC-MS Method (2):**

Table 2: HPLC: Agilent 1200 series with DA- and MS-Detector

| Method | | | **Method (2)** | |
|---|---|---|---|---|
| Column: | | | Sunfire, 3 x 30 mm, 2.5 µm | |
| | | | Waters | |
| time [min] | % Sol [$H_2O$,0.1%TFA] | %Sol [acetonitril] | flow [ml/min] | temp [°C] |
| 0.00 | 97 | 3 | 2.2 | 60 |
| 0.20 | 97 | 3 | 2.2 | 60 |
| 1.20 | 0 | 100 | 2.2 | 60 |
| 1.25 | 0 | 100 | 3 | 60 |
| 1.40 | 0 | 100 | 3 | 60 |

**Preparative Chromatography Method for Sephadex LH20 column:**

Sephadex LH20 column material is available by Sigma Life Science, LH20100-50g,

Dry LH20 powder is pre-swollen in pure methanol (4 mL methanol/g powder) at room temperature for at least 24 h. The column (dimension: 1.8 cm x 24 cm) is packed by filling the slurry into it, the gel is settled down by gravity.

Mobile phase: methanol, flow: ca. 0.7 mL/min. (gravity, no detector).

**Preparation:**

**Compound (c):**

biotin-PEG4-$NH_2$ (a) (200 mg; 0.43 mmol), (BOC-aminooxy)acetic acid (b) (83 mg; 0.43 mmol) and N-methyl-

morpholine (437 mg; 476 μl; 4.32 mmol) are suspended in dichloromethane (5 ml) and 50% 1-propanephosphonic acid cyclic anhydride solution in ethyl acetate (825 mg, 765 μl; 1.3 mmol) is added. The reaction is heated for 2 h at 40°C. The solution is diluted with dichloromethane and washed with water. The organic phase is separated and dried over an IST-Phase-Separator, the organic solvent is removed by distillation. The crude product is dissolved in methanol/acetonitrile and purified by preparative HPLC, Method (1), detection at 210 nm. The fractions of pure compound (c) are pooled and the solvent was removed by evaporation. Yield: 107 mg (39%). (M+H)+: 636; (M-H)-:634

**Compound (d):**

Compound (c) (100 mg; 0.16 mmol) is dissolved in dichloromethane (2 ml), and trifluoroacetic acid (0.5 ml; 6.48 mmol) is added. The mixture is stirred at room temperature overnight. The solution is adjusted to a basic pH by addition of triethylamine, and compound (d) is purified by preparative HPLC Method (1), detection at 215 nm. All fractions containing compound (d) are collected, and solvents are removed by distillation. Yield: 34 mg (40%); (M+H)+: 536; (M-H)-: 534

**Compound (f):**

Adenosine-5'-diphosphoribose sodium salt [compound (e)] (38 mg, 0.061 mmol) is placed in a microwave glass vial and a solution of compound (d) in methanol (1.3 ml) and sodium acetate in acetic acid (c =0.5 mol/l, 1.3 ml), is added under stirring. The tube is capped and the mixture is heated at 25-30°C for 4 h. The solution is dissolved with acetic acid, frozen and the solvent is removed by lyophilization yielding 135 mg crude compound (f) as white powder.

40 mg of crude compound (f) are dissolved in 200μl of acetic acid and 200 μl of methanol and purified by chromatography on Sephadex LH20 (see above), collection: 0.5 ml fractions in Eppendorf caps without detection. Purity of the fractions is checked by HPLC-MS Method (2). Fractions containing compound are pooled, diluted with water, frozen and the solvent is removed by lyophilization. Yield: 10.7 mg white powder (16%); (M+Na+H)++: 550; (M+H)+: 1077; (M+Na)+: 1099; H-NMR(400MHz, 1HDMSO-d6): E/Z-Isomers, purity (NMR) > 95 Mol%; HPLC (method (2)): rt: 0.646min., (M+2H)++:539

**Example 3:**

**Nucleotide ligand binding assays**

Nucleotide binding assays were performed using isothermal calorimetry (ITC) on a $ITC_{200}$ (Microcal) at 25°C. Protein was buffer exchanged using PD10 columns (GE Healthcare) into 50 mM Tris pH 7.5; 150 mM NaCl and 1 mM TCEP. Nucleotides were resuspended in ITC buffer and their concentration were determined through absorbance at 260 nm. Macro-domain proteins were used at 50 μM concentration, whereas the nucleotides were used at 500 μM. ITC measurements were performed by 20 successive injections of 2 μl of the nucleotides into 280 μl of AF1521 with a duration of 4 seconds and 150 seconds of spacing. Data analysis was done using the software ORIGIN 7.0 (Microcal).

Results:

Binding of biotinylated ADP-ribose to GST-tagged AF1521

ITC determination of the binding thermodynamics of biotinylated ADP-ribose to GST-tagged AF1521 (shown in Fig.7) and below in table 3.

Table 3 contains the analysis of two sets of data. N is the molar ratio of the interaction, $K_D$ is the equilibrium dissociation constant, ΔH is the change in enthalpy, ΔS is the change in entropy, ΔG is the change in Gibbs free energy of binding, T is the absolute temperature. The binding of biotinylated ADP-ribose to GST-tagged AF1521 is a weak interaction with a $K_D$ value of 2.5 μM and mainly driven by enthalpy.

Table 3:

| N | $K_D$ [nM] | ΔH [cal/mol] | ΔS [cal/mol/K] | ΔG [cal/mol] | -TΔS [cal/mol] |
|---|---|---|---|---|---|
| 0.77 | 2551 | 8028 | -1,3 | -7640 | 383 |

Binding of acetyl ADP-ribose to GST-tagged AF1521
Binding is shown in Fig.8 and below in table 4.

Table 4:

| N | $K_D$ [nM] | ΔH [cal/mol] | ΔS [cal/mol/K] | ΔG [cal/mol] | -TΔS [cal/mol] |
|---|---|---|---|---|---|
| 1.15 | 4142 | -5070 | 7,63 | -7339 | -2271 |

**Example 4:**

**Standard operating Procedure for screening of potential SIRT modulators**

200 nl of test compound in 100% DMSO (final concentration of compound 10 μM; DMSO 1%) is given in the 384-well plates by a Cybio Cybiwell capillary head pipettor.

After that 2 μl of an acetylated peptide (final concentration of 10 μM) mixed with $NAD^+$ (final concentration of 500 μM, dissolved in assay buffer 1) and 2 μl of SIRT enzyme (final concentration of 20 nM for 1x SIRT (100% CTL) and 200 nM for 10x SIRT (1000% CTL), dissolved in assay buffer 1) are added by a Multidrop combi. After an incubation time of 20 min at room temperature 4 μl biotinylated ADP-ribose (final concentration 50 nM, dissolved in assay buffer 2) and 4 μl GST tagged macro-domain protein (final concentration 50 nM, dissolved in assay buffer 2) are added by a Multidrop combi. After another incubation time of 60 min at room temperature 4 μl of acceptor beads and 4 μl of donor beads (final concentration of each bead-type 20 μg/ml, both dissolved in assay buffer 2) are added by a Multidrop combi. The plates are then sealed and incubated in the dark at room temperature for at least 30 min. After the incubation period, the plates are measured using the Envision reader from Perkin Elmer.

Each assay micro-titer plate contains a ADP-ribose 2'/3'-O-acetyl-ADP-ribose standard curve (100 pM, 1 nM, 10 nM, 30 nM, 100 nM, 1 μM, 3 μM, 10 μM, 100 μM) in duplicates used as reference to calculate the amount of 2'/3'-O-acetyl-ADP-ribose produced in every well.

Assay buffer 1

50 mM Tris, 150 mM NaCl, 1 mM DTT, 0.05% BSA, pH 7.5

Assay buffer 2

50 mM Tris, 150 mM NaCl, 1 mM TCEP, 0.1% Tween 20, 0.05% BSA, pH 7.5

Each assay microtiter plate contains wells with vehicle control instead of compound (1% DMSO) and 1x SIRT as reference for basic enzyme activity (100% CTL; low values) and wells with 10x SIRT as reference fully deacetylated peptide (1000% CTL; high values).

The analysis of the data is performed by the following equation:

$$\%\ CTL = 100 + ((\text{max effect }\%-100)\ (\text{high-x/high-low}))$$

Materials:

| | |
|---|---|
| 384-well small volume plates, white | → purchased from Greiner (cat.no. 784075) |
| SIRT enzyme | → prepared inhouse (see below) |
| Acetylated peptide | → purchased external |
| $NAD^+$ | → purchased from Calbiochem (cat no. 481915) |
| biotin ADP-ribose | → prepared inhouse (according to Example 2) |
| Macro-domain-GST | → prepared inhouse (according to Example 1) |
| Anti-GST acceptor beads | → purchased from Perkin Elmer |
| Streptavidin-coated donor beads | (cat no. 6760603R) |

All other materials were of highest grade commercially available.

SIRT1 enzyme preparation:

The cDNA corresponding to amino acids 183 -664 of human SIRT1 with a C-terminal 6 His-tag was synthetized and cloned into pET24a vector with NdeI and EcoRI.

The expression vector was transformed in BL21 Star (DE3) and expression was induced by 1mM IPTG at OD 0.6 and proceeded at 16°C for 20 hours.

Cells were lysed in 50 mM Tris pH 8.0., 300 mM NaCl, 1 mM TCEP and protease inhibitors (Complete EDTA-free, Roche) and incubated with Ni-NTA matrix (Qiagen) for 1 h at 4°C. The bound protein was washed with buffer containing 20 mM Imidazole, before the protein was eluated in buffer containing 250 mM Imidazole. The eluate was dialysed into 50 mM Tris pH 8.0, 50 mM NaCl and 1 mM TCEP before applied to a MonoQ column (GE Healthcare). SIRT1 protein was eluted with a salt gradient from 50-500 mM NaCl. SIRT1 containing fractions were pooled and loaded on to a gel filtration column (Superdex200, GE Healthcare) equilibrated in 25 mM Tris pH 7.5, 500 mM NaCl and 1 mM TCEP. SIRT1 containing fractions were pooled and concentrated to 3.5 mg/ml. Protein was frozen in aliquots with 10 % glycerol. SIRT1 purified by this protocol has a purity > 90 %.

SIRT1 amino acid sequence: SIRT1 aa183-664_6His

MGPYTFVQQHLMIGTDPRTILKDLLPETIPPPELDDMTLWQIVINILSEPPKRKKRKDINTIEDAVKLLQECK
KIIVLTGAGVSVSCGIPDFRSRDGIYARLAVDFPDLPDPQAMFDIEYFRKDPRPFFKFAKEIYPGQFQPSLCH
KFIALSDKEGKLLRNYTQNIDTLEQVAGIQRIIQCHGSFATASCLICKYKVDCEAVRGDIFNQVVPRCPRCP
ADEPLAIMKPEIVFFGENLPEQFHRAMKYDKDEVDLLIVIGSSLKVRPVALIPSSIPHEVPQILINREPLPHLH
FDVELLGDCDVIINELCHRLGGEYAKLCCNPVKLSEITEKPPRTQKELAYLSELPPTPLHVSEDSSSPERTSPP
DSSVIVTLLDQAAKSNDDLDVSESKGCMEEKPQEVQTSRNVESIAEQMENPDLKNVGSSTGEKNERTSV
AGTVRKCWPNRVAKEQISRRLDGNQYLFLPPNRYIFHGAEVYSDSEDDHHHHHH (SEQ ID NO: 10)

Molecular weight: 55289 Da

**<u>Example 5:</u>**

**Inhibition of 10x SIRT with 10μM EX00001253**

Assay was performed as described in example 4 (general SOP). EX00001253 reduces the amount of generated 2'/3'-O-acetyl-ADP-ribose as seen in the shift from 10x SIRT standard reaction to 10x SIRT inhibited with EX00001253 shown in Figure 4. This inhibitor is described as an antagonist for SIRT enzymes and therefor proofs the functionality of this detection system.

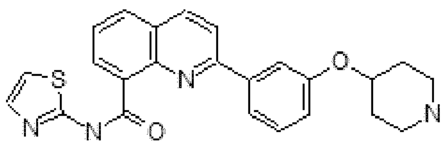

**EX00001253 known as compound 131 from** WO 2009/134973 **page 157**

**Example 6:**

**Inhibition of 10x SIRT with 10μM Suramin**

Assay was performed as described in example 4 (general SOP). Suramin reduces the amount of generated 2'/3'-O-acetyl-ADP-ribose as seen in the shift from 10x SIRT standard reaction to 10x SIRT inhibited with Suramin shown in Figure 5. This inhibitor is described as an antagonist for SIRT enzymes and therefor proves the functionality of this detection system.

**Example 7:**

**Titration curve with 2'/3'-O-acetyl-ADP-ribose**

Assay was performed as described in example 4 (general SOP) besides the fact that instead of ADP-ribose 2'/3'-O-acetyl-ADP-ribose was used for the titration curve. As shown in Fig.1 the data show the detection range under these conditions for 2'/3'-O-acetyl-ADP-ribose and underlines the functionality of the assay system for detecting different amounts of 2'/3'-O-acetyl-ADP-ribose.

**Example 8:**

**Titration curve with ADP-ribose**

Assay was performed as described in example 4 (general SOP) employing non-biotinylated ADP-ribose for the titration curve. As shown in Fig.2 the data show the detection range under these conditions for ADP-ribose and underlines the functionality of the assay system for detecting different amounts of ADP-ribose.

**Example 9:**

**Comparison of % CTL values of different compounds between a MS based Assay and a Macro-Domain Assay**

Assay was performed as described in example 5 (general SOP). Different compounds were tested using 10 μM final compound concentrations. The amount of produced 2'/3'-O-acetyl-ADP-ribose was quantified and the 1x and 10x SIRT concentrations on each assay plate were used as low (100% CTL) and high (1000% CTL) controls for calculating the % CTL values of stimulation for different compounds. In the same way an MS-based assay was performed and analyzed. The data underline that employing the high throughput compatible invented assay system generates the same data set as an MS based system.

**Table 5:**

| Compound | MS-Assay %CTL | MD-Assay %CTL |
|---|---|---|
| Compound 1 | 352 | 347 |
| Compound 2 | 220 | 217 |
| Compound 3 | 310 | 298 |
| Compound 4 | 763 | 753 |
| Compound 5 | 646 | 730 |
| Compound 6 | 385 | 357 |
| Compound 7 | 555 | 587 |
| Compound 8 | 361 | 327 |

## Claims

1. A method for measuring the activity of an enzyme, whereby the enzyme catalyzes an enzymatic reaction in which either (ia) O-acetyl-ADP-ribose is formed, or (ib) ADP-ribose or O-acetyl-ADP-ribose is used as a substrate by the enzyme, the method comprising the steps of:

   (ia) incubating the enzyme with its substrate(s) such that O-acetyl-ADP-ribose is formed,
   or
   (ib) incubating the enzyme with ADP-ribose or O-acetyl-ADP-ribose, which is then used as substrate by the enzyme,
   and
   (ii) adding to step (ia) or step (ib) a macro-domain protein and a ADP-ribose or O-acetyl-ADP-ribose, whereby the macro-domain protein comprises an acceptor complex or is bound to an acceptor complex in a further step and the ADP-ribose or O-acetyl-ADP-ribose comprises a donor complex or is bound to a donor complex in a further step, or vice versa, and
   whereby the ADP-ribose or O-acetyl-ADP-ribose competes with the ADP-ribose or O-acetyl-ADP-ribose of step (ia) or step (ib) for binding to the macro-domain protein, and
   whereby the donor complex comprises a molecule that can be excited and the acceptor complex comprises a molecule that can be stimulated by the excited donor complex, if both are in close proximity to each other, and
   (iii) exciting the mixture of step (ii), and
   (iv) measuring the signal arising upon excitation.

2. A method for identifying potential modulators of an enzyme, whereby the enzyme catalyzes an enzymatic reaction

in which either (ia) O-acetyl-ADP-ribose is formed, or (ib) ADP-ribose or O-acetyl-ADP-ribose is used as a substrate by the enzyme, the method comprising the steps of:

(ia) performing the steps of (ia)-(iv) of claim 1, whereby the potential modulator of the enzyme is added before or during any of steps (ia)-(iv),
or
(ib) performing the steps of (ib)-(iv) of claim 1, whereby the potential modulator of the enzyme is added before or during any of steps (ib)-(iv),
and
(iia) wherein after performing the steps of (ia)-(iv), a reduction or the absence of the signal obtained in the presence of the modulator is indicative for the agonistic properties of the modulator and wherein an increase of the signal in the presence of the modulator is indicative for the antagonistic properties of the modulator,
or
(iib) wherein after performing the steps of (ib)-(iv), a reduction or the absence of the signal obtained in the presence of the modulator is indicative for the agonistic properties of the modulator and wherein an increase of the signal in the presence of the modulator is indicative for the antagonistic properties of the modulator.

**3.** The method of claim 1 or 2, whereby the enzyme is a $NAD^+$ dependent protein deacetylases.

**4.** The method of claim 3, whereby the $NAD^+$ dependent protein deacetylases belongs to the class of Sirtuins.

**5.** The method of claim 1 or 2, whereby the enzyme belongs to the family of Nudix hydrolases.

**6.** The method of claim 1-2, whereby the acceptor complex and the donor complex comprises a bead.

**7.** The method of claim 6, whereby the acceptor complex and the donor complex form a luminescent proximity detection system.

**8.** The method of claim 1 or 2, whereby the O-acetyl-ADP-ribose is a 2'-O-acetyl-ADP-ribose, 3'-O-acetyl-ADP-ribose or a mixture thereof.

**9.** The method of any of the preceding claims, whereby the macro-domain protein comprises a sequence as set forth in SEQ ID NO:1 or a sequence that is at least 50%, 55%, 60%, 65%, 70%, 75% ,80%, 85%, 90%, 95% or 99% identical to that sequence.

**10.** The method of claim 1 to 8, whereby the macro-domain protein comprises the amino acids 184-370 of the sequence as set forth in SEQ ID NO:2 or a sequence that is at least 50%, 55%, 60%, 65%, 70%, 75% ,80%, 85%, 90%, 95% or 99% identical to that sequence.

**11.** The method of claim 10, whereby the macro-domain protein comprises a sequence as set forth in SEQ ID NO:2 or a sequence that is at least 50%, 55%, 60%, 65%, 70%, 75% ,80%, 85%, 90%, 95% or 99% identical to that sequence.

**12.** The method according to claim 1, comprising the steps of:

(ia) incubating a Sirtuin with an acetylated peptide substrate and $NAD^+$ such that O-acetyl-ADP-ribose is formed, and
(ii) adding to the product(s) of step (ia) a GST-tagged macro-domain protein and a biotin-tagged ADP-ribose, and adding in a further step an anti-GST acceptor bead and a streptavidin coated donor bead, whereby the anti-GST acceptor bead binds the GST-tagged macro-domain protein and the streptavidin coated donor bead binds the biotin-tagged ADP-ribose, and
(iii) exciting the mixture of step (ii) with a light source, and
(iv) measuring the signal arising upon excitation.

**13.** A kit for measuring the activity of an enzyme, whereby the enzyme catalyzes an enzymatic reaction in which either (ia) O-acetyl-ADP-ribose is formed, or (ib) ADP-ribose or O-acetyl-ADP-ribose is used as a substrate by the enzyme, comprising

(i) a macro-domain protein and a ADP-ribose or O-acetyl-ADP-ribose, and

(ii) an acceptor and a donor complex, and
(iii) (optionally) substrate(s) for the enzyme.

**14.** Use of a macro-domain protein, and either ADP-ribose or O-acetyl-ADP-ribose, and an acceptor complex and a donor complex to measure the activity of an enzyme, whereby the enzyme catalyzes an enzymatic reaction in which either O-acetyl-ADP-ribose is formed or ADP-ribose or O-acetyl-ADP-ribose is used as a substrate by the enzyme.

Figure 1

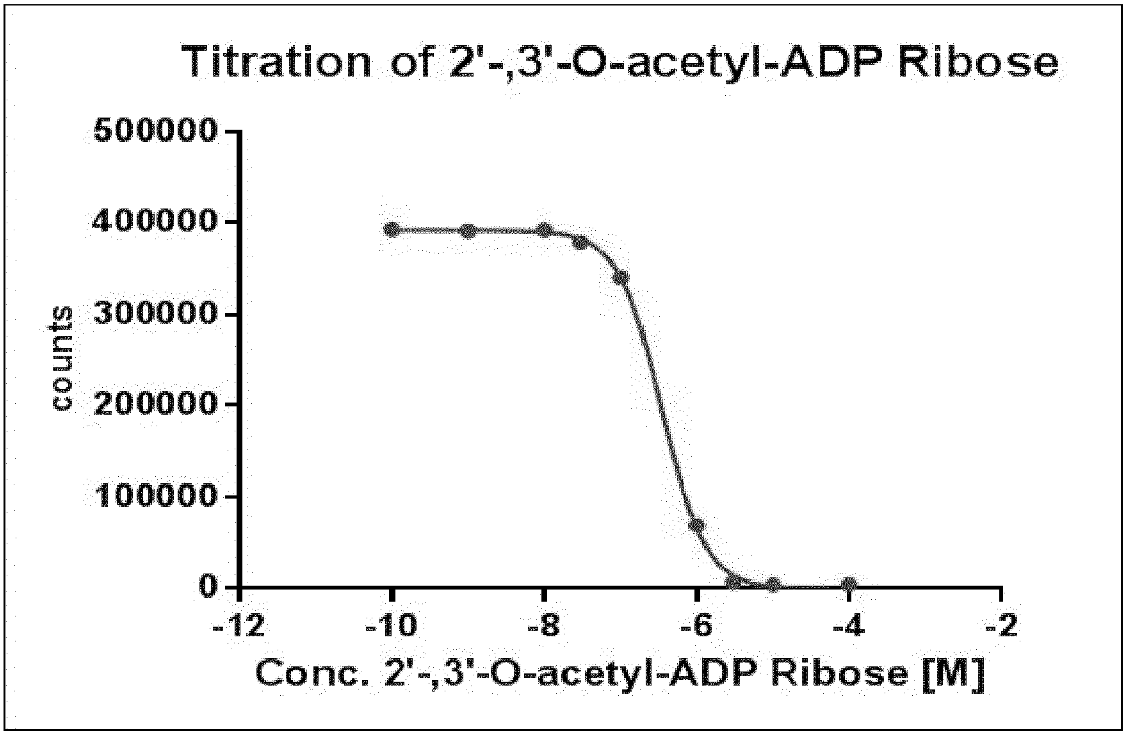
Titration of 2'-,3'-O-acetyl-ADP Ribose
500000
400000
300000
200000
100000
0
counts
-12
-10
-8
-6
-4
-2
Conc. 2'-,3'-O-acetyl-ADP Ribose [M]

Figure 2

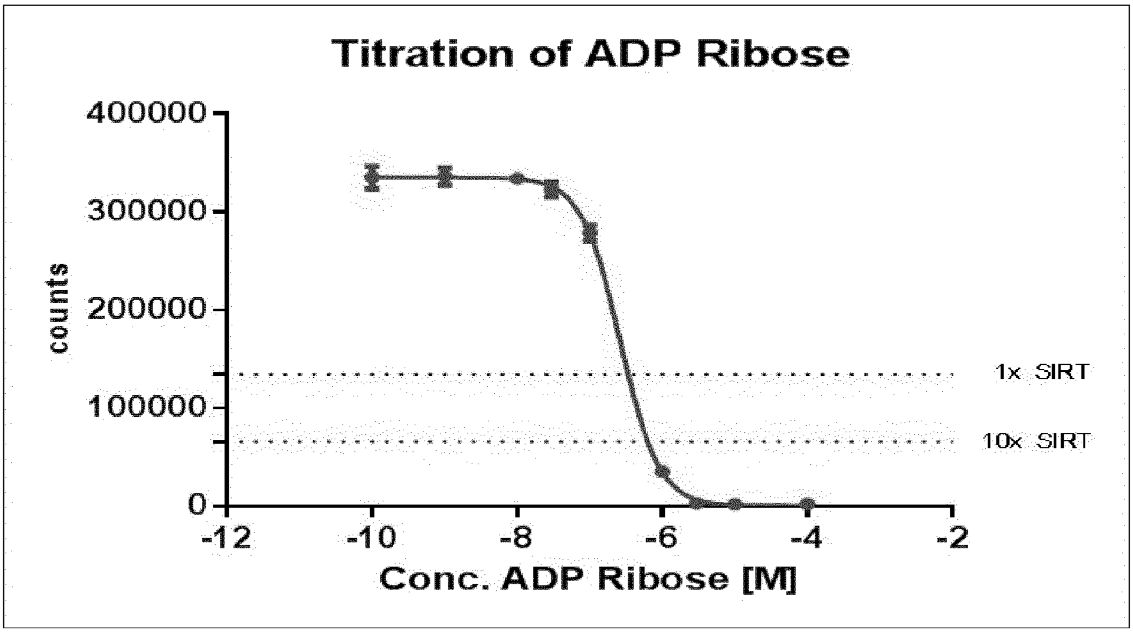
Titration of ADP Ribose
400000
300000
200000
100000
0
counts
-12
-10
-8
-6
-4
-2
Conc. ADP Ribose [M]
1x SIRT
10x SIRT

Figure 3

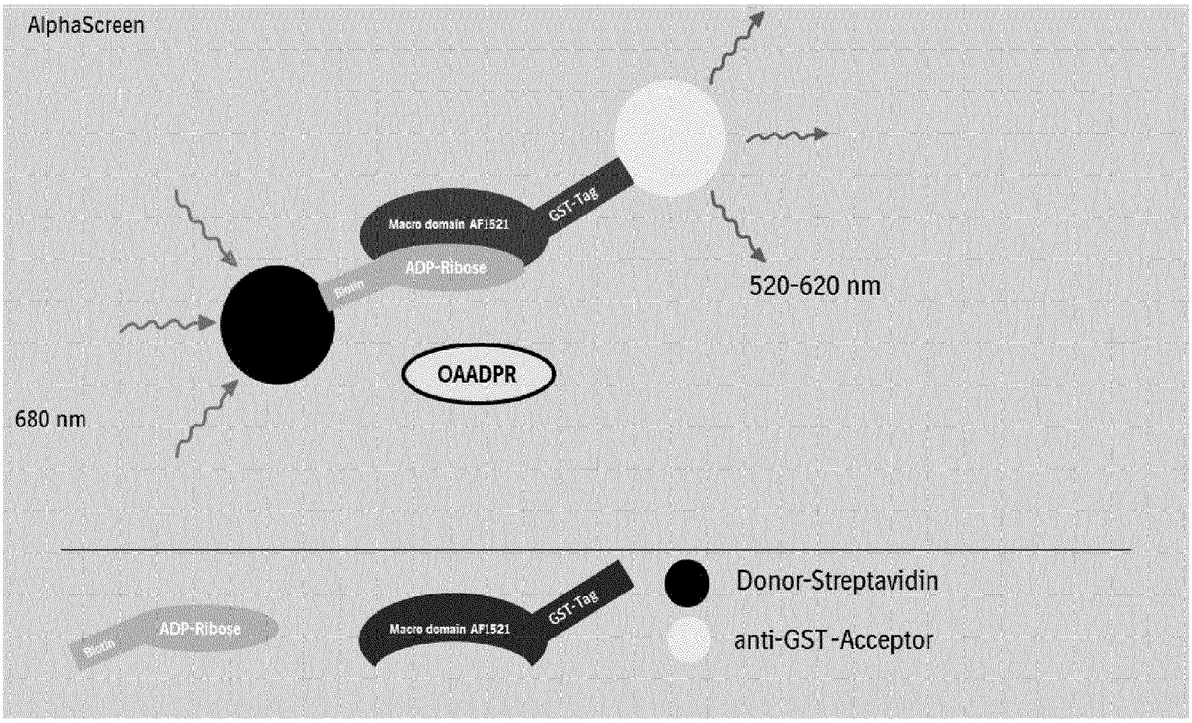
AlphaScreen
Macro domain AF1521
GST-Tag
ADP-Ribose
Biotin
520-620 nm
OAADPR
680 nm
Biotin
ADP-Ribose
Macro domain AF1521
GST-Tag
Donor-Streptavidin
anti-GST-Acceptor

Figure 4

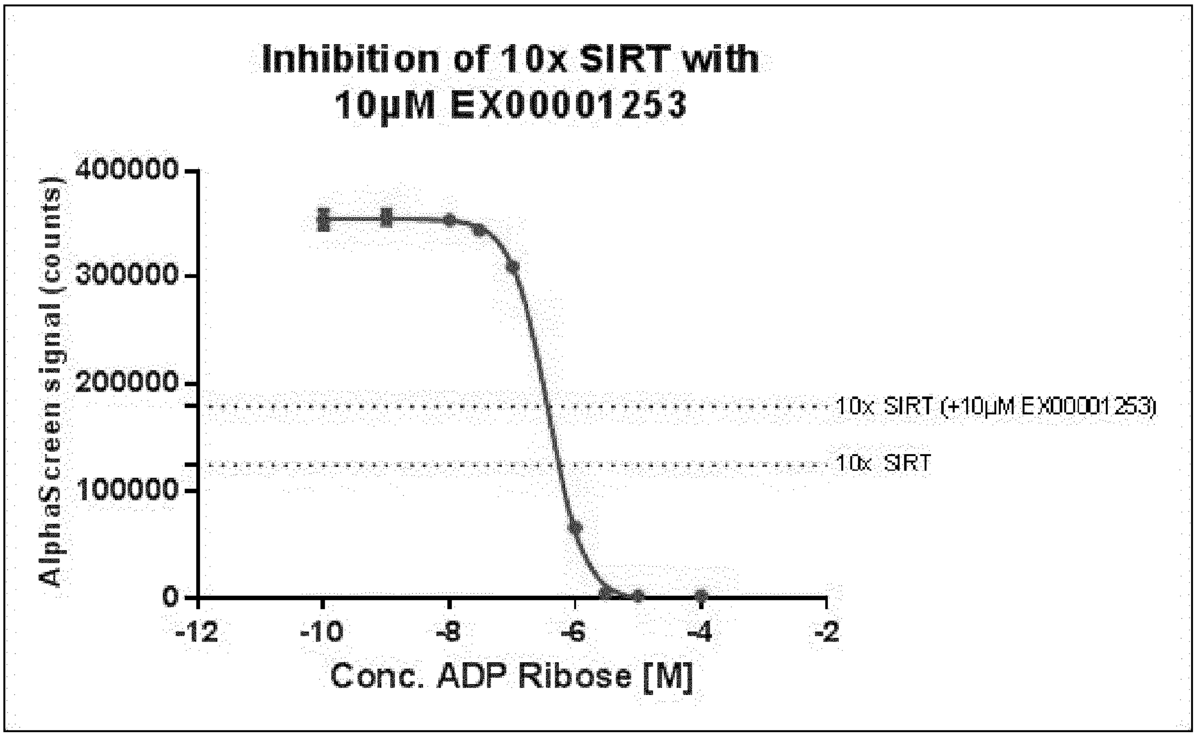
Inhibition of 10x SIRT with
10µM EX00001253
AlphaScreen signal (counts)
400000
300000
200000
100000
0
-12
-10
-8
-6
-4
-2
Conc. ADP Ribose [M]
10x SIRT (+10µM EX00001253)
10x SIRT

Figure 5

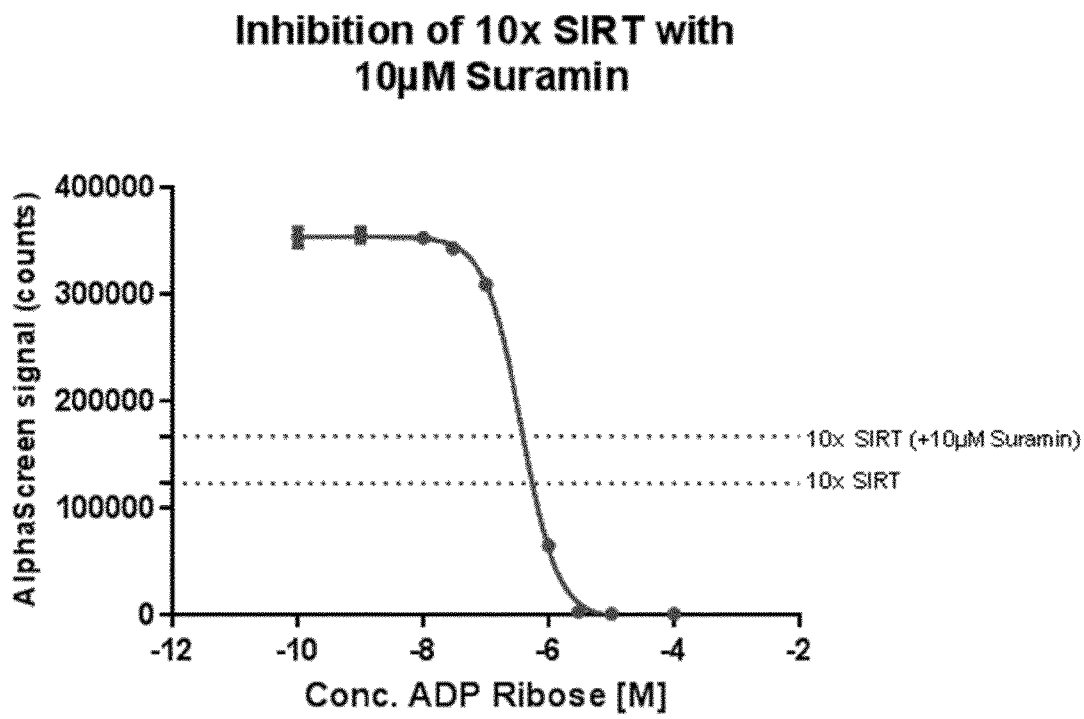
Inhibition of 10x SIRT with
10µM Suramin
AlphaScreen signal (counts)
400000
300000
200000
100000
0
-12
-10
-8
-6
-4
-2
Conc. ADP Ribose [M]
10x SIRT (+10µM Suramin)
10x SIRT

Figure 6

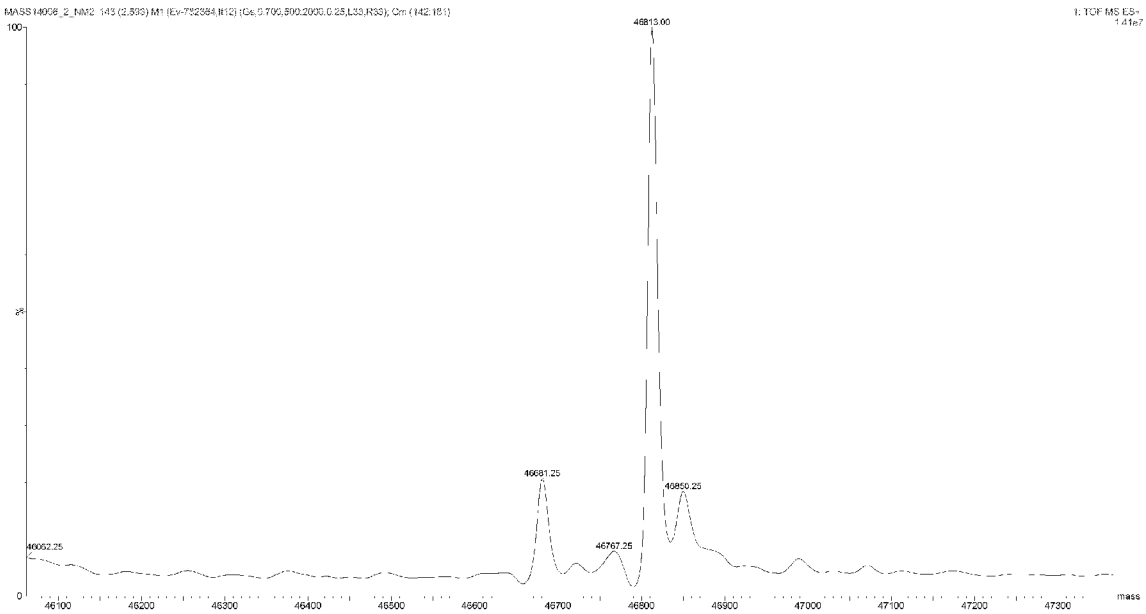
MASS14006_2_NM2 143 (2.593) M1 (Ev-752364,It12) (Gs,0.700,500:2000,0.25,L33,R33); Cm (142:181)
1: TOF MS ES+
1.41e7
100
%
0
46813.00
46681.25
46850.25
46767.25
46062.25
46100
46200
46300
46400
46500
46600
46700
46800
46900
47000
47100
47200
47300
mass

Figure 7

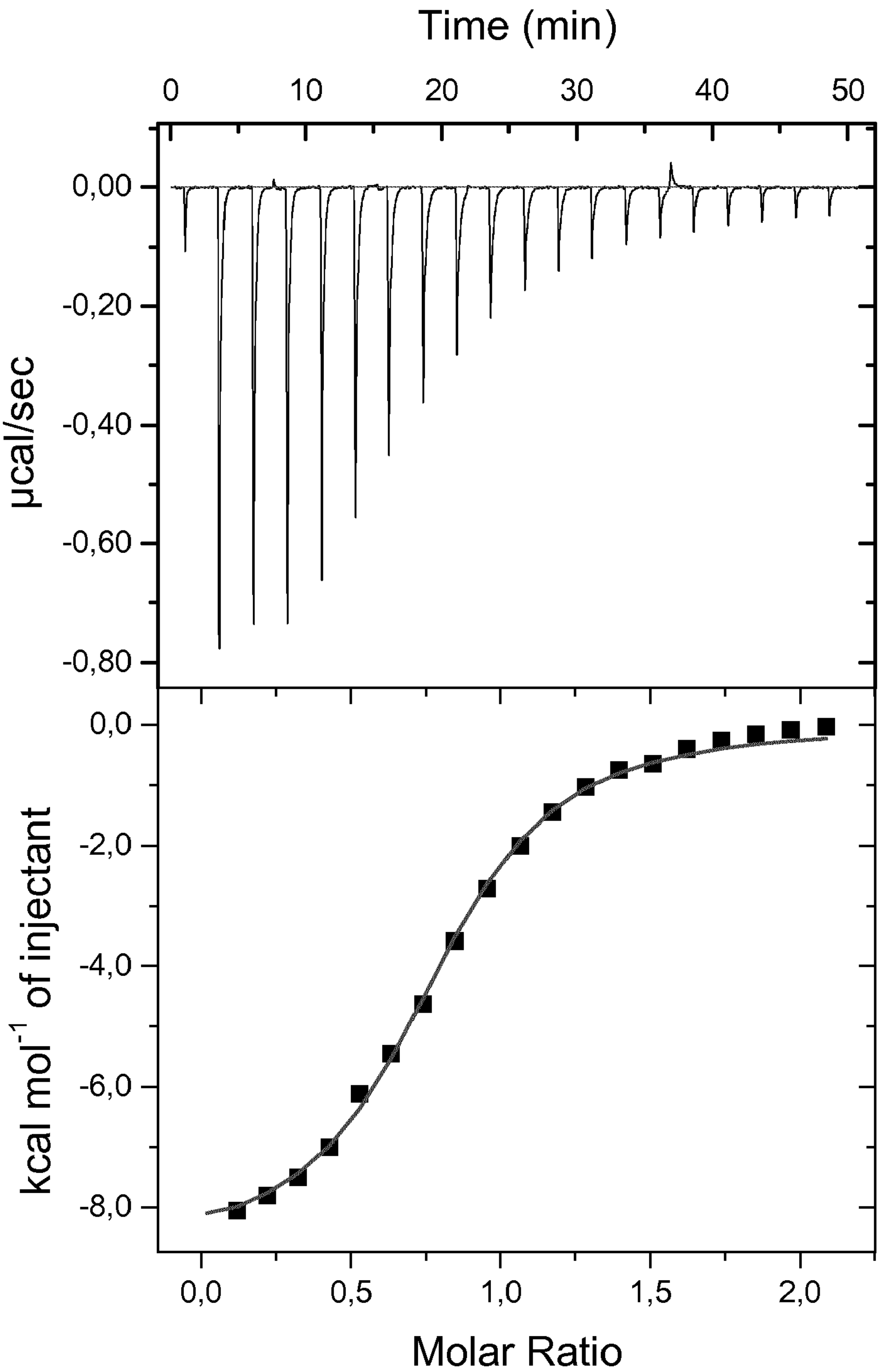
Time (min)
0
10
20
30
40
50
0,00
-0,20
-0,40
-0,60
-0,80
µcal/sec
0,0
-2,0
-4,0
-6,0
-8,0
kcal mol-1 of injectant
0,0
0,5
1,0
1,5
2,0
Molar Ratio

Figure 8

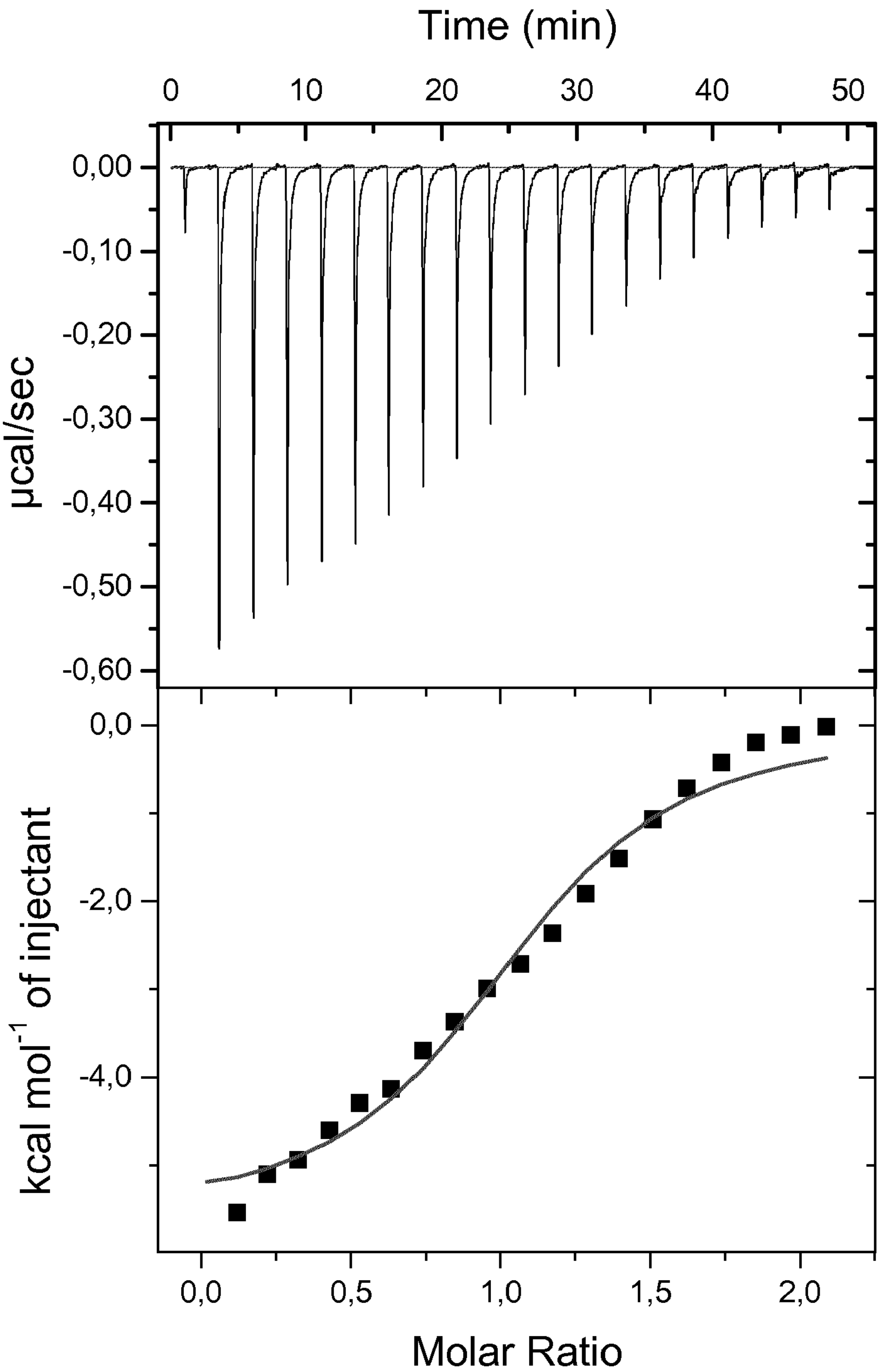
Time (min)
0
10
20
30
40
50
0,00
-0,10
-0,20
-0,30
-0,40
-0,50
-0,60
µcal/sec
0,0
-2,0
-4,0
kcal mol-1 of injectant
0,0
0,5
1,0
1,5
2,0
Molar Ratio

Europäisches Patentamt
European Patent Office
Office européen des brevets

# EUROPEAN SEARCH REPORT

Application Number
EP 15 16 2317

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | DOMINY JOHN ET AL: "In Vivo Measurement of the Acetylation State of Sirtuin Substrates as a Proxy for Sirtuin Activity", SIRTUINS: METHODS AND PROTOCOLS, vol. 1077, 2013, pages 217-237, XP008177678, DOI: 10.1007/978-1-62703-637-5_15 * the whole document * ----- | 1-14 | INV. G01N33/50 C12Q1/34 |
| X | NORTH B J ET AL: "Preparation of enzymatically active recombinant class III protein deacetylases", METHODS, vol. 36, no. 4, 1 August 2005 (2005-08-01), pages 338-345, XP027216546, ACADEMIC PRESS ISSN: 1046-2023 [retrieved on 2005-08-19] * the whole document * ----- | 1-3,5-14 | |
| A | LANDRY J ET AL: "Enzymatic assays for NAD-dependent deacetylase activities", METHODS, vol. 31, no. 1, 1 September 2003 (2003-09-01), pages 33-39, XP004441569, ACADEMIC PRESS ISSN: 1046-2023, DOI: 10.1016/S1046-2023(03)00085-9 * the whole document * ----- | 1-14 | TECHNICAL FIELDS SEARCHED (IPC) G01N C12Q |

The present search report has been drawn up for all claims

3

| Place of search | Date of completion of the search | Examiner |
|---|---|---|
| The Hague | 13 October 2015 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009134973 A


### Non-patent literature cited in the description


- *Expert Opin. Drug Discov.,* 2014, vol. 9 (2), 183-199
- *Analytical Biochemistry,* 2004, vol. 332, 90-99
- *Journal of Biomolecular Screening,* 2011, vol. 16 (10), 1227-1235
- *Anal. Biochem.,* 2009, vol. 394, 101-109
- *Analytical Biochemistry,* 2009, vol. 395, 205-210
- *Analytical Biochemistry,* 2008, vol. 378, 53-59
- *J. Biol. Chem.,* 2005, vol. 280, 17187-17195
- *J. Biol. Chem.,* 2005, vol. 280, 17038-17045
- *Anal. Biochem.,* 2009, vol. 395, 205-210
- *Anal. Biochem.,* 2008, vol. 378, 53-59
- *Anal Biochem.,* 2008, vol. 383 (29), 174-179
- *Journal of Biomolecular Screening,* 2011, vol. 16 (10), 1217-1226
- *J. Am. Chem. Soc.,* 2010, vol. 132, 15878

SEQUENCE LISTING

<110> Boehringer Ingelheim International GmbH

<120> Detection and quantification of O-acetyl-ADP-ribose as a readout system for measuring the activity of e.g. Sirtuins

<130> P01-3107/EP/1

<160> 10

<170> BiSSAP 1.2

<210> 1
<211> 192
<212> PRT
<213> Archaeoglobus fulgidus

<400> 1
```
Met Glu Val Leu Phe Glu Ala Lys Val Gly Asp Ile Thr Leu Lys Leu
1               5                   10                  15
Ala Gln Gly Asp Ile Thr Gln Tyr Pro Ala Lys Ala Ile Val Asn Ala
            20                  25                  30
Ala Asn Lys Arg Leu Glu His Gly Gly Gly Val Ala Tyr Ala Ile Ala
        35                  40                  45
Lys Ala Cys Ala Gly Asp Ala Gly Leu Tyr Thr Glu Ile Ser Lys Lys
    50                  55                  60
Ala Met Arg Glu Gln Phe Gly Arg Asp Tyr Ile Asp His Gly Glu Val
65                  70                  75                  80
Val Val Thr Pro Ala Met Asn Leu Glu Glu Arg Gly Ile Lys Tyr Val
                85                  90                  95
Phe His Thr Val Gly Pro Ile Cys Ser Gly Met Trp Ser Glu Glu Leu
            100                 105                 110
Lys Glu Lys Leu Tyr Lys Ala Phe Leu Gly Pro Leu Glu Lys Ala Glu
        115                 120                 125
Glu Met Gly Val Glu Ser Ile Ala Phe Pro Ala Val Ser Ala Gly Ile
    130                 135                 140
Tyr Gly Cys Asp Leu Glu Lys Val Val Glu Thr Phe Leu Glu Ala Val
145                 150                 155                 160
Lys Asn Phe Lys Gly Ser Ala Val Lys Glu Val Ala Leu Val Ile Tyr
                165                 170                 175
Asp Arg Lys Ser Ala Glu Val Ala Leu Lys Val Phe Glu Arg Ser Leu
            180                 185                 190
```

<210> 2
<211> 372
<212> PRT
<213> Homo sapiens

<400> 2
```
Met Ser Ser Arg Gly Gly Lys Lys Lys Ser Thr Lys Thr Ser Arg Ser
1               5                   10                  15
Ala Lys Ala Gly Val Ile Phe Pro Val Gly Arg Met Leu Arg Tyr Ile
            20                  25                  30
Lys Lys Gly His Pro Lys Tyr Arg Ile Gly Val Gly Ala Pro Val Tyr
        35                  40                  45
Met Ala Ala Val Leu Glu Tyr Leu Thr Ala Glu Ile Leu Glu Leu Ala
    50                  55                  60
Gly Asn Ala Ala Arg Asp Asn Lys Lys Gly Arg Val Thr Pro Arg His
```

```
65                  70                  75                  80
Ile Leu Leu Ala Val Ala Asn Asp Glu Glu Leu Asn Gln Leu Leu Lys
                85                  90                  95
Gly Val Thr Ile Ala Ser Gly Gly Val Leu Pro Asn Ile His Pro Glu
            100                 105                 110
Leu Leu Ala Lys Lys Arg Gly Ser Lys Gly Lys Leu Glu Ala Ile Ile
        115                 120                 125
Thr Pro Pro Pro Ala Lys Lys Ala Lys Ser Pro Ser Gln Lys Lys Pro
    130                 135                 140
Val Ser Lys Lys Ala Gly Gly Lys Lys Gly Ala Arg Lys Ser Lys Lys
145                 150                 155                 160
Lys Gln Gly Glu Val Ser Lys Ala Ala Ser Ala Asp Ser Thr Thr Glu
                165                 170                 175
Gly Thr Pro Ala Asp Gly Phe Thr Val Leu Ser Thr Lys Ser Leu Phe
            180                 185                 190
Leu Gly Gln Lys Leu Asn Leu Ile His Ser Glu Ile Ser Asn Leu Ala
        195                 200                 205
Gly Phe Glu Val Glu Ala Ile Ile Asn Pro Thr Asn Ala Asp Ile Asp
    210                 215                 220
Leu Lys Asp Asp Leu Gly Asn Thr Leu Glu Lys Lys Gly Gly Lys Glu
225                 230                 235                 240
Phe Val Glu Ala Val Leu Glu Leu Arg Lys Lys Asn Gly Pro Leu Glu
                245                 250                 255
Val Ala Gly Ala Ala Val Ser Ala Gly His Gly Leu Pro Ala Lys Phe
            260                 265                 270
Val Ile His Cys Asn Ser Pro Val Trp Gly Ala Asp Lys Cys Glu Glu
        275                 280                 285
Leu Leu Glu Lys Thr Val Lys Asn Cys Leu Ala Leu Ala Asp Asp Lys
    290                 295                 300
Lys Leu Lys Ser Ile Ala Phe Pro Ser Ile Gly Ser Gly Arg Asn Gly
305                 310                 315                 320
Phe Pro Lys Gln Thr Ala Ala Gln Leu Ile Leu Lys Ala Ile Ser Ser
                325                 330                 335
Tyr Phe Val Ser Thr Met Ser Ser Ser Ile Lys Thr Val Tyr Phe Val
            340                 345                 350
Leu Phe Asp Ser Glu Ser Ile Gly Ile Tyr Val Gln Glu Met Ala Lys
        355                 360                 365
Leu Asp Ala Asn
    370
```

<210> 3
<211> 193
<212> PRT
<213> Artificial Sequence

<220>
<223> AF1521_N-His_cleaved

<400> 3

```
Gly Met Glu Val Leu Phe Glu Ala Lys Val Gly Asp Ile Thr Leu Lys
1               5                   10                  15
Leu Ala Gln Gly Asp Ile Thr Gln Tyr Pro Ala Lys Ala Ile Val Asn
            20                  25                  30
Ala Ala Asn Lys Arg Leu Glu His Gly Gly Gly Val Ala Tyr Ala Ile
        35                  40                  45
Ala Lys Ala Cys Ala Gly Asp Ala Gly Leu Tyr Thr Glu Ile Ser Lys
    50                  55                  60
Lys Ala Met Arg Glu Gln Phe Gly Arg Asp Tyr Ile Asp His Gly Glu
65                  70                  75                  80
Val Val Val Thr Pro Ala Met Asn Leu Glu Glu Arg Gly Ile Lys Tyr
                85                  90                  95
Val Phe His Thr Val Gly Pro Ile Cys Ser Gly Met Trp Ser Glu Glu
            100                 105                 110
```

```
Leu Lys Glu Lys Leu Tyr Lys Ala Phe Leu Gly Pro Leu Glu Lys Ala
        115                 120                 125
Glu Glu Met Gly Val Glu Ser Ile Ala Phe Pro Ala Val Ser Ala Gly
    130                 135                 140
Ile Tyr Gly Cys Asp Leu Glu Lys Val Val Glu Thr Phe Leu Glu Ala
145                 150                 155                 160
Val Lys Asn Phe Lys Gly Ser Ala Val Lys Glu Val Ala Leu Val Ile
                165                 170                 175
Tyr Asp Arg Lys Ser Ala Glu Val Ala Leu Lys Val Phe Glu Arg Ser
            180                 185                 190
Leu


<210> 4
<211> 225
<212> PRT
<213> Artificial Sequence

<220>
<223> AF1521_N-His

<400> 4
Met Ala Ser Met Thr Gly Gly Gln Gln Met Gly Arg Gly Ser Met Gly
1               5                   10                  15
Ser His His His His His His Ser Ser Gly Glu Asn Leu Tyr Phe Gln
            20                  25                  30
Gly Met Glu Val Leu Phe Glu Ala Lys Val Gly Asp Ile Thr Leu Lys
        35                  40                  45
Leu Ala Gln Gly Asp Ile Thr Gln Tyr Pro Ala Lys Ala Ile Val Asn
    50                  55                  60
Ala Ala Asn Lys Arg Leu Glu His Gly Gly Gly Val Ala Tyr Ala Ile
65                  70                  75                  80
Ala Lys Ala Cys Ala Gly Asp Ala Gly Leu Tyr Thr Glu Ile Ser Lys
                85                  90                  95
Lys Ala Met Arg Glu Gln Phe Gly Arg Asp Tyr Ile Asp His Gly Glu
            100                 105                 110
Val Val Val Thr Pro Ala Met Asn Leu Glu Glu Arg Gly Ile Lys Tyr
        115                 120                 125
Val Phe His Thr Val Gly Pro Ile Cys Ser Gly Met Trp Ser Glu Glu
    130                 135                 140
Leu Lys Glu Lys Leu Tyr Lys Ala Phe Leu Gly Pro Leu Glu Lys Ala
145                 150                 155                 160
Glu Glu Met Gly Val Glu Ser Ile Ala Phe Pro Ala Val Ser Ala Gly
                165                 170                 175
Ile Tyr Gly Cys Asp Leu Glu Lys Val Val Glu Thr Phe Leu Glu Ala
            180                 185                 190
Val Lys Asn Phe Lys Gly Ser Ala Val Lys Glu Val Ala Leu Val Ile
        195                 200                 205
Tyr Asp Arg Lys Ser Ala Glu Val Ala Leu Lys Val Phe Glu Arg Ser
    210                 215                 220
Leu
225

<210> 5
<211> 338
<212> PRT
<213> Artificial Sequence

<220>
<223> AF1521_N-GST

<400> 5
Met Leu Gly Gly Cys Pro Lys Glu Arg Ala Glu Ile Ser Met Leu Glu
```

```
1               5                   10                  15
Gly Ala Val Leu Asp Ile Arg Tyr Gly Val Ser Arg Ile Ala Tyr Ser
            20                  25                  30
Lys Asp Phe Glu Thr Leu Lys Val Asp Phe Leu Ser Lys Leu Pro Glu
        35                  40                  45
Met Leu Lys Met Phe Glu Asp Arg Leu Cys His Lys Thr Tyr Leu Asn
    50                  55                  60
Gly Asp His Val Thr His Pro Asp Phe Met Leu Tyr Asp Ala Leu Asp
65                  70                  75                  80
Val Val Leu Tyr Met Asp Pro Met Cys Leu Asp Ala Phe Pro Lys Leu
                85                  90                  95
Val Cys Phe Lys Lys Arg Ile Glu Ala Ile Pro Gln Ile Asp Lys Tyr
            100                 105                 110
Leu Lys Ser Ser Lys Tyr Ile Ala Trp Pro Leu Gln Gly Trp Gln Ala
        115                 120                 125
Thr Phe Gly Gly Gly Asp His Pro Pro Lys Ser Asp Leu Val Pro Arg
    130                 135                 140
Gly Ser Met Glu Val Leu Phe Glu Ala Lys Val Gly Asp Ile Thr Leu
145                 150                 155                 160
Lys Leu Ala Gln Gly Asp Ile Thr Gln Tyr Pro Ala Lys Ala Ile Val
                165                 170                 175
Asn Ala Ala Asn Lys Arg Leu Glu His Gly Gly Gly Val Ala Tyr Ala
            180                 185                 190
Ile Ala Lys Ala Cys Ala Gly Asp Ala Gly Leu Tyr Thr Glu Ile Ser
        195                 200                 205
Lys Lys Ala Met Arg Glu Gln Phe Gly Arg Asp Tyr Ile Asp His Gly
    210                 215                 220
Glu Val Val Val Thr Pro Ala Met Asn Leu Glu Glu Arg Gly Ile Lys
225                 230                 235                 240
Tyr Val Phe His Thr Val Gly Pro Ile Cys Ser Gly Met Trp Ser Glu
                245                 250                 255
Glu Leu Lys Glu Lys Leu Tyr Lys Ala Phe Leu Gly Pro Leu Glu Lys
            260                 265                 270
Ala Glu Glu Met Gly Val Glu Ser Ile Ala Phe Pro Ala Val Ser Ala
        275                 280                 285
Gly Ile Tyr Gly Cys Asp Leu Glu Lys Val Val Glu Thr Phe Leu Glu
    290                 295                 300
Ala Val Lys Asn Phe Lys Gly Ser Ala Val Lys Glu Val Ala Leu Val
305                 310                 315                 320
Ile Tyr Asp Arg Lys Ser Ala Glu Val Ala Leu Lys Val Phe Glu Arg
                325                 330                 335
Ser Leu


<210> 6
<211> 229
<212> PRT
<213> Artificial Sequence

<220>
<223> AF1521_N-Strep

<400> 6
Met Trp Ser His Pro Gln Phe Glu Lys Gly Gly Gly Ser Gly Gly Gly
1               5                   10                  15
Ser Gly Gly Ser Ala Trp Ser His Pro Gln Phe Glu Lys Gly Ser Glu
            20                  25                  30
Asn Leu Tyr Phe Gln Met Glu Val Leu Phe Glu Ala Lys Val Gly Asp
        35                  40                  45
Ile Thr Leu Lys Leu Ala Gln Gly Asp Ile Thr Gln Tyr Pro Ala Lys
    50                  55                  60
Ala Ile Val Asn Ala Ala Asn Lys Arg Leu Glu His Gly Gly Gly Val
65                  70                  75                  80
```

```
Ala Tyr Ala Ile Ala Lys Ala Cys Ala Gly Asp Ala Gly Leu Tyr Thr
                85                  90                  95
Glu Ile Ser Lys Lys Ala Met Arg Glu Gln Phe Gly Arg Asp Tyr Ile
            100                 105                 110
Asp His Gly Glu Val Val Val Thr Pro Ala Met Asn Leu Glu Glu Arg
        115                 120                 125
Gly Ile Lys Tyr Val Phe His Thr Val Gly Pro Ile Cys Ser Gly Met
    130                 135                 140
Trp Ser Glu Glu Leu Lys Glu Lys Leu Tyr Lys Ala Phe Leu Gly Pro
145                 150                 155                 160
Leu Glu Lys Ala Glu Glu Met Gly Val Glu Ser Ile Ala Phe Pro Ala
                165                 170                 175
Val Ser Ala Gly Ile Tyr Gly Cys Asp Leu Glu Lys Val Val Glu Thr
            180                 185                 190
Phe Leu Glu Ala Val Lys Asn Phe Lys Gly Ser Ala Val Lys Glu Val
        195                 200                 205
Ala Leu Val Ile Tyr Asp Arg Lys Ser Ala Glu Val Ala Leu Lys Val
    210                 215                 220
Phe Glu Arg Ser Leu
225
```

<210> 7
<211> 209
<212> PRT
<213> Artificial Sequence

<220>
<223> H2AY_N-HisTEV

<400> 7

```
Met Gly Ser His His His His His His Ser Ser Gly Glu Asn Leu Tyr
1               5                   10                  15
Phe Gln Gly Phe Thr Val Leu Ser Thr Lys Ser Leu Phe Leu Gly Gln
            20                  25                  30
Lys Leu Asn Leu Ile His Ser Glu Ile Ser Asn Leu Ala Gly Phe Glu
        35                  40                  45
Val Glu Ala Ile Ile Asn Pro Thr Asn Ala Asp Ile Asp Leu Lys Asp
    50                  55                  60
Asp Leu Gly Asn Thr Leu Glu Lys Lys Gly Gly Lys Glu Phe Val Glu
65                  70                  75                  80
Ala Val Leu Glu Leu Arg Lys Lys Asn Gly Pro Leu Glu Val Ala Gly
                85                  90                  95
Ala Ala Val Ser Ala Gly His Gly Leu Pro Ala Lys Phe Val Ile His
            100                 105                 110
Cys Asn Ser Pro Val Trp Gly Ala Asp Lys Cys Glu Glu Leu Leu Glu
        115                 120                 125
Lys Thr Val Lys Asn Cys Leu Ala Leu Ala Asp Asp Lys Lys Leu Lys
    130                 135                 140
Ser Ile Ala Phe Pro Ser Ile Gly Ser Gly Arg Asn Gly Phe Pro Lys
145                 150                 155                 160
Gln Thr Ala Ala Gln Leu Ile Leu Lys Ala Ile Ser Ser Tyr Phe Val
                165                 170                 175
Ser Thr Met Ser Ser Ser Ile Lys Thr Val Tyr Phe Val Leu Phe Asp
            180                 185                 190
Ser Glu Ser Ile Gly Ile Tyr Val Gln Glu Met Ala Lys Leu Asp Ala
        195                 200                 205
Asn
```

<210> 8
<211> 228
<212> PRT
<213> Artificial Sequence

<220>
<223> H2AY_N-Strep

<400> 8

```
Met Trp Ser His Pro Gln Phe Glu Lys Gly Gly Gly Ser Gly Gly Gly
1               5                   10                  15
Ser Gly Gly Ser Ala Trp Ser His Pro Gln Phe Glu Lys Gly Ser Glu
            20                  25                  30
Asn Leu Tyr Phe Gln Gly Phe Thr Val Leu Ser Thr Lys Ser Leu Phe
        35                  40                  45
Leu Gly Gln Lys Leu Asn Leu Ile His Ser Glu Ile Ser Asn Leu Ala
    50                  55                  60
Gly Phe Glu Val Glu Ala Ile Ile Asn Pro Thr Asn Ala Asp Ile Asp
65                  70                  75                  80
Leu Lys Asp Asp Leu Gly Asn Thr Leu Glu Lys Lys Gly Gly Lys Glu
                85                  90                  95
Phe Val Glu Ala Val Leu Glu Leu Arg Lys Lys Asn Gly Pro Leu Glu
            100                 105                 110
Val Ala Gly Ala Ala Val Ser Ala Gly His Gly Leu Pro Ala Lys Phe
        115                 120                 125
Val Ile His Cys Asn Ser Pro Val Trp Gly Ala Asp Lys Cys Glu Glu
    130                 135                 140
Leu Leu Glu Lys Thr Val Lys Asn Cys Leu Ala Leu Ala Asp Asp Lys
145                 150                 155                 160
Lys Leu Lys Ser Ile Ala Phe Pro Ser Ile Gly Ser Gly Arg Asn Gly
                165                 170                 175
Phe Pro Lys Gln Thr Ala Ala Gln Leu Ile Leu Lys Ala Ile Ser Ser
            180                 185                 190
Tyr Phe Val Ser Thr Met Ser Ser Ser Ile Lys Thr Val Tyr Phe Val
        195                 200                 205
Leu Phe Asp Ser Glu Ser Ile Gly Ile Tyr Val Gln Glu Met Ala Lys
    210                 215                 220
Leu Asp Ala Asn
225
```

<210> 9
<211> 337
<212> PRT
<213> Artificial Sequence

<220>
<223> H2AY_N-GST

<400> 9

```
Met Leu Gly Gly Cys Pro Lys Glu Arg Ala Glu Ile Ser Met Leu Glu
1               5                   10                  15
Gly Ala Val Leu Asp Ile Arg Tyr Gly Val Ser Arg Ile Ala Tyr Ser
            20                  25                  30
Lys Asp Phe Glu Thr Leu Lys Val Asp Phe Leu Ser Lys Leu Pro Glu
        35                  40                  45
Met Leu Lys Met Phe Glu Asp Arg Leu Cys His Lys Thr Tyr Leu Asn
    50                  55                  60
Gly Asp His Val Thr His Pro Asp Phe Met Leu Tyr Asp Ala Leu Asp
65                  70                  75                  80
Val Val Leu Tyr Met Asp Pro Met Cys Leu Asp Ala Phe Pro Lys Leu
                85                  90                  95
Val Cys Phe Lys Lys Arg Ile Glu Ala Ile Pro Gln Ile Asp Lys Tyr
            100                 105                 110
Leu Lys Ser Ser Lys Tyr Ile Ala Trp Pro Leu Gln Gly Trp Gln Ala
        115                 120                 125
Thr Phe Gly Gly Gly Asp His Pro Pro Lys Ser Asp Leu Val Pro Arg
    130                 135                 140
```

```
Gly Ser Gly Phe Thr Val Leu Ser Thr Lys Ser Leu Phe Leu Gly Gln
145                 150                 155                 160
Lys Leu Asn Leu Ile His Ser Glu Ile Ser Asn Leu Ala Gly Phe Glu
                165                 170                 175
Val Glu Ala Ile Ile Asn Pro Thr Asn Ala Asp Ile Asp Leu Lys Asp
            180                 185                 190
Asp Leu Gly Asn Thr Leu Glu Lys Lys Gly Gly Lys Glu Phe Val Glu
        195                 200                 205
Ala Val Leu Glu Leu Arg Lys Lys Asn Gly Pro Leu Glu Val Ala Gly
    210                 215                 220
Ala Ala Val Ser Ala Gly His Gly Leu Pro Ala Lys Phe Val Ile His
225                 230                 235                 240
Cys Asn Ser Pro Val Trp Gly Ala Asp Lys Cys Glu Glu Leu Leu Glu
                245                 250                 255
Lys Thr Val Lys Asn Cys Leu Ala Leu Ala Asp Asp Lys Lys Leu Lys
            260                 265                 270
Ser Ile Ala Phe Pro Ser Ile Gly Ser Gly Arg Asn Gly Phe Pro Lys
        275                 280                 285
Gln Thr Ala Ala Gln Leu Ile Leu Lys Ala Ile Ser Ser Tyr Phe Val
    290                 295                 300
Ser Thr Met Ser Ser Ser Ile Lys Thr Val Tyr Phe Val Leu Phe Asp
305                 310                 315                 320
Ser Glu Ser Ile Gly Ile Tyr Val Gln Glu Met Ala Lys Leu Asp Ala
                325                 330                 335
Asn


<210> 10
<211> 489
<212> PRT
<213> Artificial Sequence

<220>
<223> SIRT1 amino acid sequence: SIRT1 aa183-664_6His

<400> 10
Met Gly Pro Tyr Thr Phe Val Gln Gln His Leu Met Ile Gly Thr Asp
1               5                   10                  15
Pro Arg Thr Ile Leu Lys Asp Leu Leu Pro Glu Thr Ile Pro Pro Pro
            20                  25                  30
Glu Leu Asp Asp Met Thr Leu Trp Gln Ile Val Ile Asn Ile Leu Ser
        35                  40                  45
Glu Pro Pro Lys Arg Lys Lys Arg Lys Asp Ile Asn Thr Ile Glu Asp
    50                  55                  60
Ala Val Lys Leu Leu Gln Glu Cys Lys Lys Ile Ile Val Leu Thr Gly
65                  70                  75                  80
Ala Gly Val Ser Val Ser Cys Gly Ile Pro Asp Phe Arg Ser Arg Asp
                85                  90                  95
Gly Ile Tyr Ala Arg Leu Ala Val Asp Phe Pro Asp Leu Pro Asp Pro
            100                 105                 110
Gln Ala Met Phe Asp Ile Glu Tyr Phe Arg Lys Asp Pro Arg Pro Phe
        115                 120                 125
Phe Lys Phe Ala Lys Glu Ile Tyr Pro Gly Gln Phe Gln Pro Ser Leu
    130                 135                 140
Cys His Lys Phe Ile Ala Leu Ser Asp Lys Glu Gly Lys Leu Leu Arg
145                 150                 155                 160
Asn Tyr Thr Gln Asn Ile Asp Thr Leu Glu Gln Val Ala Gly Ile Gln
                165                 170                 175
Arg Ile Ile Gln Cys His Gly Ser Phe Ala Thr Ala Ser Cys Leu Ile
            180                 185                 190
Cys Lys Tyr Lys Val Asp Cys Glu Ala Val Arg Gly Asp Ile Phe Asn
        195                 200                 205
Gln Val Val Pro Arg Cys Pro Arg Cys Pro Ala Asp Glu Pro Leu Ala
```

```
    210                 215                 220
Ile Met Lys Pro Glu Ile Val Phe Phe Gly Glu Asn Leu Pro Glu Gln
225                 230                 235                 240
Phe His Arg Ala Met Lys Tyr Asp Lys Asp Glu Val Asp Leu Leu Ile
                245                 250                 255
Val Ile Gly Ser Ser Leu Lys Val Arg Pro Val Ala Leu Ile Pro Ser
            260                 265                 270
Ser Ile Pro His Glu Val Pro Gln Ile Leu Ile Asn Arg Glu Pro Leu
        275                 280                 285
Pro His Leu His Phe Asp Val Glu Leu Leu Gly Asp Cys Asp Val Ile
    290                 295                 300
Ile Asn Glu Leu Cys His Arg Leu Gly Gly Glu Tyr Ala Lys Leu Cys
305                 310                 315                 320
Cys Asn Pro Val Lys Leu Ser Glu Ile Thr Glu Lys Pro Pro Arg Thr
                325                 330                 335
Gln Lys Glu Leu Ala Tyr Leu Ser Glu Leu Pro Pro Thr Pro Leu His
            340                 345                 350
Val Ser Glu Asp Ser Ser Ser Pro Glu Arg Thr Ser Pro Pro Asp Ser
        355                 360                 365
Ser Val Ile Val Thr Leu Leu Asp Gln Ala Ala Lys Ser Asn Asp Asp
    370                 375                 380
Leu Asp Val Ser Glu Ser Lys Gly Cys Met Glu Glu Lys Pro Gln Glu
385                 390                 395                 400
Val Gln Thr Ser Arg Asn Val Glu Ser Ile Ala Glu Gln Met Glu Asn
                405                 410                 415
Pro Asp Leu Lys Asn Val Gly Ser Ser Thr Gly Glu Lys Asn Glu Arg
            420                 425                 430
Thr Ser Val Ala Gly Thr Val Arg Lys Cys Trp Pro Asn Arg Val Ala
        435                 440                 445
Lys Glu Gln Ile Ser Arg Arg Leu Asp Gly Asn Gln Tyr Leu Phe Leu
    450                 455                 460
Pro Pro Asn Arg Tyr Ile Phe His Gly Ala Glu Val Tyr Ser Asp Ser
465                 470                 475                 480
Glu Asp Asp His His His His His His
                485
```